# EUROPEAN PATENT APPLICATION

(11) **EP 3 940 058 A1**
(43) Date of publication of application: **19.01.2022**
(21) Application number: 20186345.3
(22) Date of filing: 16.07.2020
(51) Int. Cl.: C12N 5/071, C12M 1/00, A01N 1/02, A61L 27/60, G01N 33/50

(54) **MICROFLUIDIC SYSTEM TO CONTROL PERFUSION, DIFFUSION AND COLLECTION OF MOLECULES OVER LONG PERIODS IN AN EX-VIVO SKIN MODEL**

(71) Applicant: Genoskin, 31100 Toulouse (FR); Centre National de la Recherche Scientifique (CNRS), 75794 Paris Cedex 16 (FR)
(72) Inventor: Descargues, Pascal, Swampscott, MA 01907 (US); Pagès, Emeline, 31320 Castanet-Tolosan (FR); Malaquin, Laurent, 31450 Ayguesvives (FR); Raude, Emma, 31300 Toulouse (FR)
(74) Representative: Barbot, Willy

(57) **Abstract**

The present invention relates to a microfluidic device implanted in an *ex-vivo* skin explant to control perfusion, diffusion and collection of molecules over long periods. The invention also relates to a method for assessing permeation or infusion of a biomarker of interest through the skin and to a method for detecting and/or quantifying a biomarker of interest contained in the liquid secreted by the *ex-vivo* skin explant.

## Description

### FIELD OF THE INVENTION

The present invention relates to a microfluidic device implanted in an *ex-vivo* skin explant to control perfusion, diffusion and collection of molecules over long periods.

### BACKGROUND OF THE INVENTION

The skin is an organ composed of many different cell types and structures with specialized functions. The main role of the skin is to act as a protective barrier between the inside and the outside of the body. Exposure of the skin to chemicals or other materials may result in a variety of pathological effects ranging from surface effects to deeper topical effects or even systemic effects if penetration through the skin occurs. When a compound manages to cross the epidermis, it becomes accessible for the dermis and potentially accessible to the systemic circulatory and lymphatic systems.

Blood flow to the skin relies on complex ultrastructure and organization of the cutaneous microvasculature. The microvessels in the papillary dermis and in the deep dermis have a range size of about 10-50 µm, whereas microvessels of up to 100 µm such as arterioles or venules are also occasionally present. The cutaneous arteries arise from the subcutaneous tissue and enter the dermis to form the cutaneous arterial plexuses. The arterioles and venules of the cutaneous microcirculation form two important horizontal plexuses parallel to the skin surface: an upper horizontal plexus in the papillary dermis (subepidermal or subpapillary plexus) from which the nutritive capillary loops arise and a lower horizontal plexus at the dermal-subcutaneous border. These two plexuses communicate with each other with arterioles and venules and represent the physiologically important areas in the skin (Irwin Braverman, 2000, J Investig Dermatol Symp Proc., vol.5(1): pp.3-9)

Apart from being essential for human thermoregulation, the skin microcirculation has to ensure nutrient delivery to the epidermis and its adnexa, and to take a major part in inflammation and wound healing.

Penetration of a potentially harmful exogenous agent though the skin barrier can trigger numerous toxic effects such as an inflammatory reaction, an allergic reaction and more dramatically a carcinogenic process. The Organization For Economic Cooperation And Development (OECD) recommends methods for the assessment of dermal penetration of compounds in *in vitro, ex vivo* and *in vivo* models.

*In vivo* studies should be conducted using OECD TG 427. Briefly, the test sample is applied on the clipped skin of animals for the desired amount of time, under occlusive, semi-occlusive or non-occlusive conditions. The animals are kept in metabolism cages in order to study the complete metabolic profile of the compound tested. However, pretreatment of the skin such as shaving, depilation and clipping commonly used to prepare skin for in vivo studies, may also affect the results obtain in terms of dermal absorption. Moreover, due to species differences in terms of skin structure, especially dermal layer thickness, lipid composition, and pelage density, differences in terms of dermal penetration/ absorption can differ markedly between animal and human skin. Lastly, more and more States concerned with the welfare of animals used for experimental and scientific purposes are banning this type of experimentation.

*In vitro*/*ex vivo* studies should be conducted using OECD TG 428. The test consists of applying a test substance onto the surface of a skin preparation that separates two chambers: donor and receptor chambers. After exposure for the desired amount of time under the chosen conditions, the test sample is cleaned from the skin and the receptor fluid is analyzed for the test compound or its metabolites. The skin preparation can be of human or animal origin. Nevertheless, human skin remains the "gold standard" for assessing the dermal penetration/absorption of compounds in humans. Reconstituted three dimensional human skin models such as EpiDerm^{®} and EpiSkin^{®} can also be used. Unfortunately, these reconstructed human epidermis were more permeable than human skin and therefore were also over estimating the dermal absorption of benchmark compounds. Moreover, reconstructed human epidermis does not have microvessels. Another important drawback is that the *in vitro* models to study dermal penetration are usually static. However, stimulation of the skin by natural movement of a living animal, or massage can influence the level of dermal penetration/ absorption of a compound.

There is therefore a need for implementing new tools to overcome these shortcomings, and in particular for obtaining human ex-vivo human skin explant which closely resemble native human skin vasculature.

### SUMMARY OF THE INVENTION

The present invention allows compensating for the above-mentioned disadvantages by providing an implantable microfluidic device allowing for the infusion and extraction of molecular species or colloidal suspensions in an *ex-vivo* model of skin.

Thus, a first object of the invention relates to an *in vitro* method for culturing an *ex-vivo* skin explant comprising:
(a) inserting an implantable microfluidic device through the dermis or hypodermis of an *ex-vivo* skin explant so that the device is positioned substantially parallel to the epidermis and traverses the skin explant from side to side, both ends of the device protruding slightly beyond the skin explant,
(b) connecting the two ends of the implantable microfluidic device to two separate tubings,
(c) immersing the skin explant obtained in step (b) in a liquid matrix capable of solidifying so that the upper surface of the epidermis is not covered, which matrix is itself contained in a cell culture insert, the bottom of which consists of a porous membrane,
(d) solidifying the matrix so as to trap the immersed part of the skin explant, where the upper surface of the epidermis is not covered, and to cause the solidified matrix to adhere to the side walls and the porous membrane of the insert,
(e) putting the culture insert containing the skin explant obtained in step (d) in a culture chamber containing appropriate culture medium, and
(f) culturing the skin explant.

Pores present in a portion of the implanted microfluidic device that passes through the *ex-vivo* skin explant have a diameter from about 10 µm to about 250 µm and allow molecules, drugs or compounds of interest to be delivered in the dermis or hypodermis of the skin explant.

In a preferred embodiment, the method of the invention further comprises a step (g) of connecting the tubings to a circulating system comprising means for providing a continuous or discontinuous/pulsatile flow and/or for modulating the difference of pressure between the inlet and the outlet of the implantable device, said connecting being mediated by input and output ports at the inlet and outlet of the implantable device.

The complete circulating system allows a control of the flow rate of a fluid perfused within the *ex-vivo* skin explant, and/or its inlet and outlet pressure.

The system obtained by the method of the invention can be connected to vials and means for delivering culture media, drugs or any molecule of interest for performing further studies, such as pharmacokinetic and/or pharmacodynamics profile of drugs.

In another preferred embodiment, the method of the invention is for further detecting and/or quantifying at least one biomarker of interest contained in the interstitial fluid of the *ex-vivo* skin explant, and said method further comprises the following steps:
(h) collecting the liquid secreted by the *ex-vivo* skin explant through the output port, and
(i) isolating, identifying, and optionally quantifying the at least one biomarker of interest contained in the collected fractions of the liquid secreted by the *ex-vivo* skin explant.

In another preferred embodiment, the method of the invention is for further assessing permeation of the at least one biomarker of interest though the skin, said method further comprises a step (g') of applying topically to the epidermis of the *ex-vivo* skin explant a composition comprising the at least one biomarker of interest after the step (g) of connecting the tubings to a circulating system and prior to the step (h) of collecting the liquid secreted by the *ex-vivo* skin explant.

In still another preferred embodiment, the method of the invention is for further assessing the activity of at least one compound intradermally injected, said method further comprises a step (g") of injecting a composition comprising the at least one compound of interest after the step (g) of connecting the tubings to a circulating system and prior to the step (h) of collecting the liquid secreted by the *ex-vivo* skin explant, the composition being injecting in the dermis of the *ex-vivo* skin explant by using the microfluidic implantable device.

In another embodiment, the method of the invention is for further assessing the activity of at least one compounds subcutaneously injected, said method further comprises a step (g'") of injecting a composition comprising the at least one compound of interest after the step (g) of connecting the tubings to a circulating system and prior to the step (h) of collecting the liquid secreted by the *ex-vivo* skin explant, the composition being injecting in the hypodermis of the *ex-vivo* skin explant by using the microfluidic implantable device.

In another embodiment, the method of the invention is for further oxygenating the ex-*vivo skin* explant, said method further comprises a step (g"") of injecting an oxygen carrier after the step (g) of connecting the tubings to a circulating system

In another preferred embodiment, the method of the invention is for further studying inflammation of the skin, said method further comprises a step (g'"") of injecting a composition comprising a cocktail of cytokines after the step (g) of connecting the tubings to a circulating system and prior to the step (h) of collecting the liquid secreted by the *ex-vivo* skin explant, the composition being injecting in the dermis or hypodermis of the *ex-vivo* skin explant by using the microfluidic implantable device.

Yet another object of the invention relates to a system comprising a cell culture insert the bottom of which consists of a porous membrane, said culture insert containing an *ex-vivo* skin explant embedded in a solidified matrix, wherein the *ex-vivo* skin explant comprises the epidermis, the dermis and optionally the hypodermis characterized in that an implantable microfluidic device is inserted through the dermal or hypodermal part of the *ex-vivo* skin explant, wherein the microfluidic device is positioned substantially parallel to the epidermis and traverses the *ex-vivo* skin explant from side to side, both ends of the device protruding slightly beyond the *ex-vivo* skin explant.

The intended uses of the system described above are the following ones:
- identifying percutaneous penetration of potentially dangerous exogenous agents from the environment,
- administering high molecular weight molecules in a time-controlled manner,
- studying the diffusion of an infused compound into the dermis or hypodermis,
- comparing different routes of administration,
- testing different modes of administration (over time),
- studying skin inflammation,
- assessing toxicity of drugs administered by subcutaneous route,
- assessing bioavailability of a cosmetic agent administered by topical route,
- assessing permeation rate of a compound administered by topical route, or
- obtaining long term culture of *ex-vivo* skin explant.

Several tools were developed to improve the reproducibility of the *ex-vivo* skin explant models obtained by the method of the invention, and trade named FlowSkin^{®}. Among them, a cell culture plate and a device used as mold for inserting the microfluidic device in the *ex-vivo* skin explant are particularly suitable for the implementation of the method of the invention.

Another object of the invention relates to a device for inserting a microfluidic implantable device into an *ex-vivo* skin explant, said device comprising two separate and removable stackable parts which, one assembled together, form:
(a) a central cavity suitable for receiving and maintaining an *ex-vivo* skin explant, and
(b) ducts through which a microfluidic implantable device is guided to go through the ex-vivo skin explant from side to side.

Finally, the invention relates to a method for inserting a microfluidic implantable device in an *ex-vivo* skin explant, comprising the steps of:
(a) placing the skin explant in the recess of the first stackable part of the device as defined above, where the epidermis is in contact with this first element and the dermis or hypodermis is in contact with the air,
(b) assembling the second stackable part of the device on the first stackable part containing the skin explant so that the dermis or hypodermis of the explant is in contact with the recess of the second stackable part of the device,
(c) fastening the two stackable parts of the device comprising the skin explant,
(d) introducing the implantable microfluidic device in the groove,
(e) passing the implantable microfluidic device through the skin explant from side to side so as to position the porous part of the implantable microfluidic device is located in the center of the skin explant.

The present invention and its preferred embodiments are described in further details below.

### BRIEF DESCRIPTION OF THE DRAWINGS

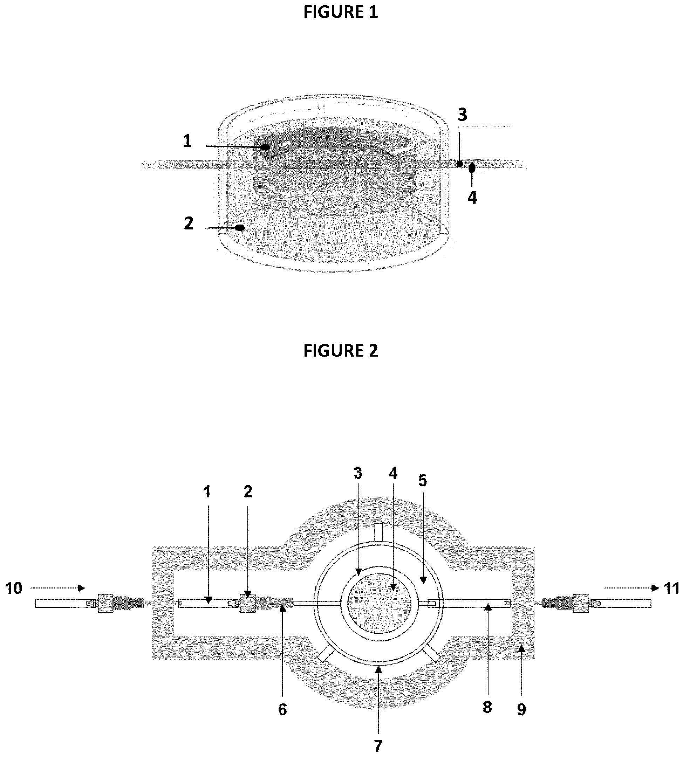

Figure 1 is an illustration of the *ex-vivo* skin explant implanted with a microfluidic device obtained by the method of the invention. A cell culture insert, the bottom of which consists of a porous membrane, contains the *ex-vivo* skin explant (1) implanted with a microfluidic device (4) containing culture medium, a drug or any other molecule of interest (3). The *ex-vivo* skin explant is embedded in a solidified matrix (2).
Figure 2 shows an exemplary system of the invention that is used in examples: tubings (1) and (8), connection element (2), ring of hydrophobic material (3), *ex-vivo* skin explant (4), solidified matrix (5), microfluidic device (6), cell culture insert (7), cell culture container (9), inlet end (10) and outlet end (11).

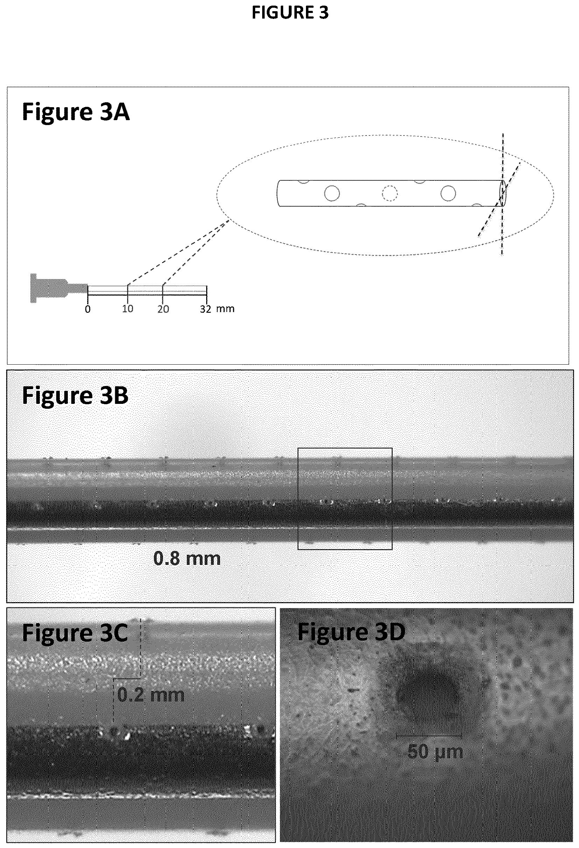

Figure 3 shows drawings and illustrations of an implantable device according to the invention.
Figure 3A is a mechanical drawing of the porous design of a part of the implantable microfluidic device.
Figure 3B depicts porous part of the catheter used as implantable microfluidic device (light microscope). A gap of approximately 0.8 mm is present between two consecutive pores aligned on one side of the implantable device.
Figure 3C is an enlarged view of the holes performed in a part of the catheter used as implantable microfluidic device (Light microscope). An offset of approximately 0.2 mm occurs between the pores of two sides of the implantable device.
Figure 3D shows a pore with a diameter of approximately 50 µm (Scanning electron microscope).

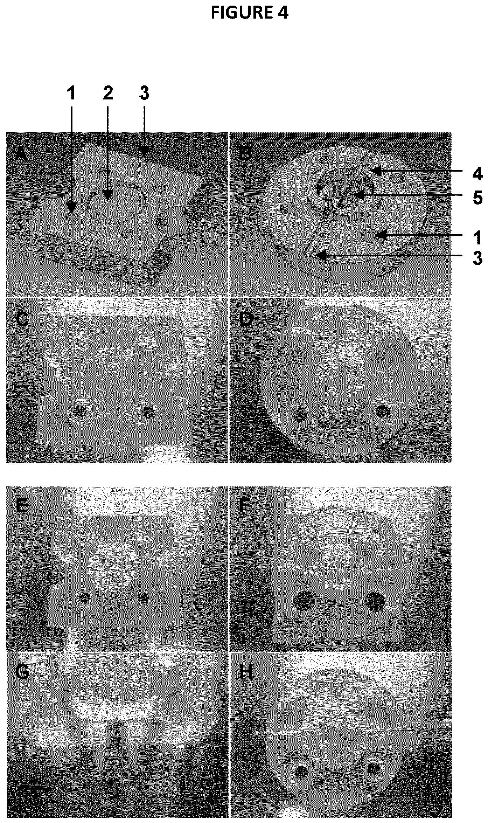

Figure 4 shows a drawing (A and B) and an illustration (C and D) of the device used for inserting a microfluidic implantable device into an *ex-vivo* skin explant. Both base parts (A) and (C) and top parts (B) and (D) of the device have grooves (3) in contact with a central recess (2). Means for preventing the *ex-vivo* skin explant from slipping of the device are present in the top part of the device (B and D), such as a crown (4) and pins (5). Both base parts (A) and (C) and top parts (B) and (D) of the device also have fastening means suitable (1), such as for example magnets, for stacking the two parts of the device. The skin explant is deposited in the recess, epidermis against the lower part (E). The upper part is stacked to the lower part, trapping the biopsy (F). A catheter is inserted into the grooves provided for this purpose (G). The two parts are separated to release the skin explant, then the catheter needle is removed (H).

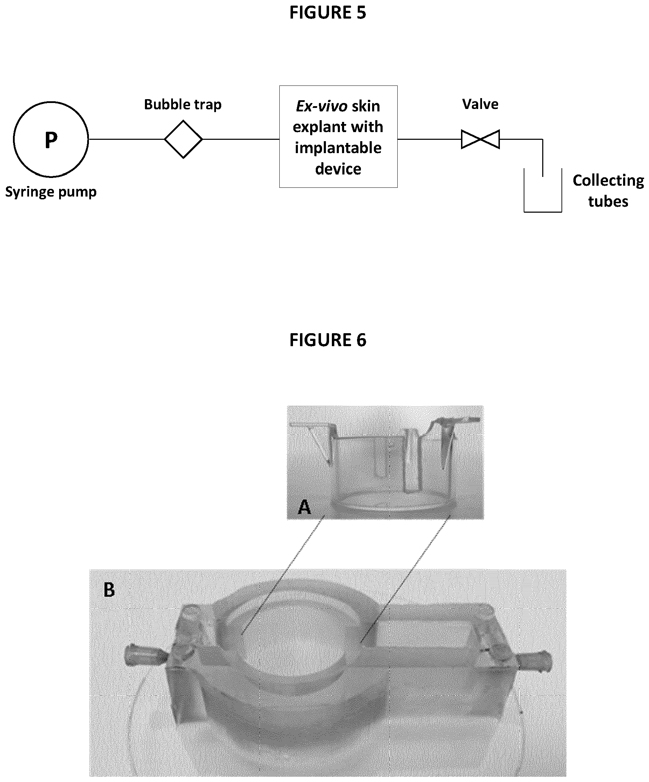

Figure 5 is a schematic view of the microfluidic system.
Figure 6 is an illustration of a culture chamber (B) and a culture insert (A) suitable for receiving an *ex-vivo* skin explant having an implantable microfluidic device through the dermis or hypodermis.

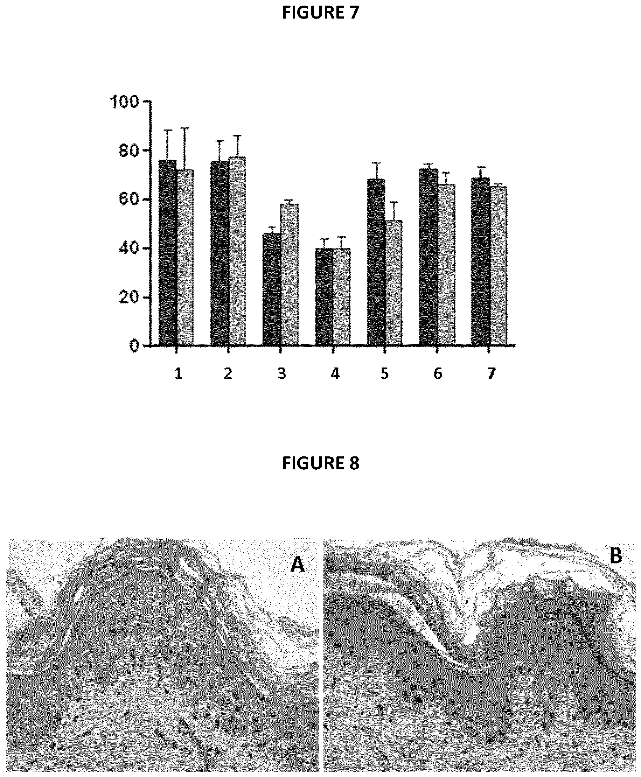

Figure 7 is the quantification of cell viability of *ex-vivo* skin explants issued from seven different donors (1 to 7) and cultured over 10 days in conditions described in the examples, in dark the NativeSkin^{®} models and in grey the FlowSkin^{®} models. Viability is measured using the Cell Counting Kit-8 (Sigma) and is expressed as the percentage of skin viability measured the day of models' production. Each bar represents means ±SEM of cell viability values measured for each condition.
Figure 8 depicts a representative image of the histological characteristics (Hematoxylin and Eosin staining) of *ex-vivo* skin explants issued from one donor and cultured over 10 days in conditions described in the examples, (A) NativeSkin^{®} model and (B) FlowSkin^{®} model.

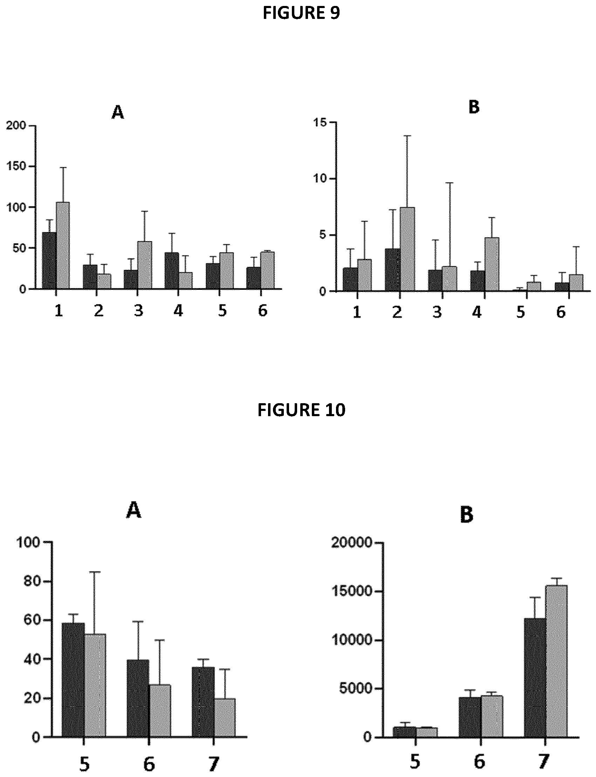

Figure 9 A is the quantification of the number of proliferating cells in the epidermis of the *ex-vivo* skin explants issued from six different donors (1, 2, 3, 4, 5 and 6) and cultured over 10 days in conditions described in the examples, NativeSkin^{®} models in dark and FlowSkin^{®} models in grey. The number of proliferating cells is assessed using an anti-Ki67 immunostaining and expressed as the percentage of proliferation measured the day of models' production. Each bar represents means ±SEM of proliferation measured for each condition.
Figure 9 B is the quantification of the percentage of apoptosis in the epidermis of the *ex-vivo* skin explants issued from six different donors (1, 2, 3, 4, 5 and 6) and cultured over 10 days in conditions described in the examples, NativeSkin^{®} models in dark and FlowSkin^{®} models in grey. The percentage of apoptosis is assessed using an anti-cleaved Caspase-3 immunostaining. Each bar represents means ±SEM of apoptosis measured for each condition.
Figure 10 is the characterization of the cell metabolic activity of the *ex-vivo* skin explants issued from three different donors (5, 6 and 7) and cultured over 10 days in conditions described in the examples, NativeSkin^{®} models in dark and FlowSkin^{®} models in grey Metabolic activity is assessed by glucose consumption (A) and lactic acid production (B). Each bar represents means ±SEM of the percentage of glucose consumed (A) or the concentration of lactic acid (µM) produced (B).

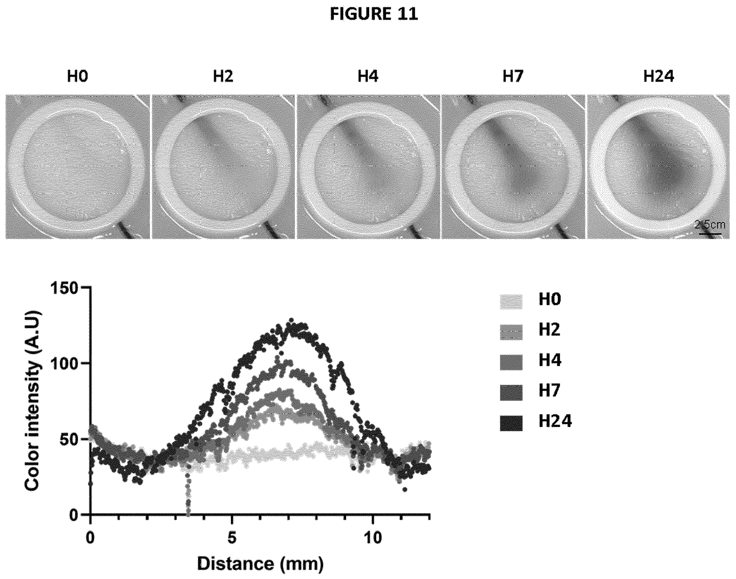

Figure 11 is illustrative of dye diffusion in the *ex-vivo* skin explants model cultured over 24 hours in conditions described in the examples. The graph represents the color intensity (arbitrary unit) - which is correlated with dye diffusion - measured following a line perpendicular to the catheter (0 cm is one edge of the biopsy and 12 mm is the other edge of the biopsy), at different time points (0, 2, 4, 7 and 24 hours of perfusion).

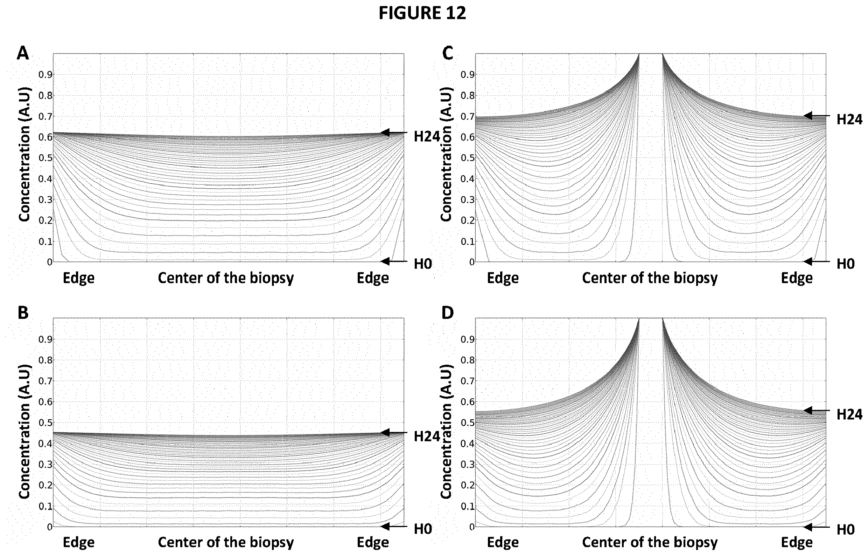

Figure 12 is a diffusion modeling in NativeSkin^{®} and FlowSkin^{®} models using COMSOL software. The graphs represent the evolution of the concentration of molecules of interest on a sectional view at the center of the *ex-vivo* skin explant, at times ranging from 0 to 24 hours of culture. A and B: NativeSkin^{®}. C and D: FlowSkin^{®}.
   A and C: Modeling the diffusion of a molecule of interest present both in the culture medium in the well under *ex-vivo* skin explant and in the perfusate at an arbitrary initial concentration set at 1, and in the matrix at a concentration of 0.4. This model can be equated to simulating the diffusion of nutrients present in the culture medium (as this culture medium is also present in the matrix).
   B and D: Modeling the diffusion of a molecule of interest present only in the well under *ex-vivo* skin explant and in the perfusate at an arbitrary initial concentration set at 1. This model can be equated to simulating the diffusion of a drug administered systemically.

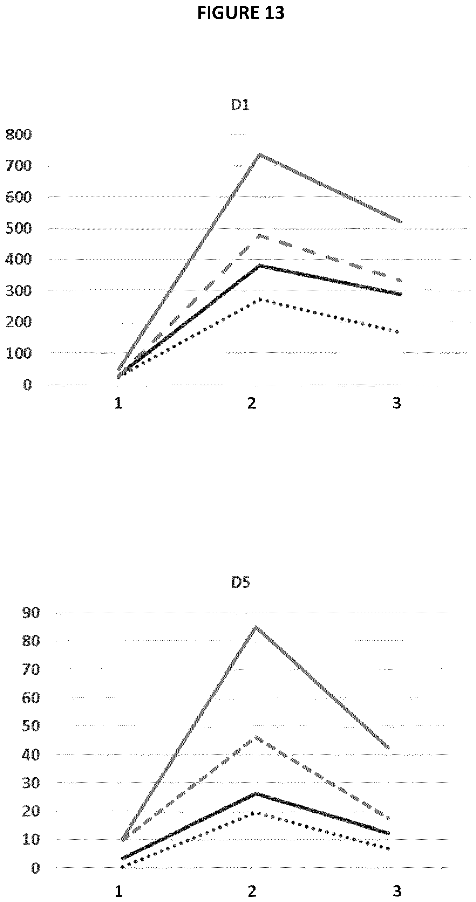

Figure 13 is a follow up of IL-22 release in the culture media of *ex-vivo* skin explants inflammatory model over 3 days. T-cell activation of *ex-vivo* skin explants issued from two donors (D1 and D5) was achieved using a cocktail of anti-CD3 and anti-CD28 antibodies. After T-cell differentiation, the *ex-vivo* skin explants were treated with a cocktail of pro-inflammatory cytokines (IL-1β + TGF-β + IL-23) mixed only in the culture media (full and doted black lines) or in the culture media and infused intradermally (full and doted grey lines) using the FlowSkin^{®} system. Cytokine concentration is expressed in pg/ml.

### DETAILED DESCRIPTION OF THE INVENTION

The inventors have now developed a new *ex-vivo* skin explant model in which an implantable microfluidic device makes it possible to control perfusion, diffusion and collection of molecules over long periods. Hence, such a modified *ex-vivo* skin explant model is suitable for the administration and study of drug absorption. In addition, the control of perfusion and diffusion of molecules over long periods may allow to increase viability of the implanted *ex-vivo* skin explant.

In a first aspect, the present invention relates to an *in-vitro* method for culturing an *ex-vivo* skin explant comprising:
(a) inserting an implantable microfluidic device through the dermis or hypodermis of an *ex-vivo* skin explant so that the device is positioned substantially parallel to the epidermis and traverses the skin explant from side to side, both ends of the device protruding slightly beyond the skin explant,
(b) connecting the two ends of the implantable microfluidic device to two separate tubings,
(c) immersing the skin explant obtained in step (b) in a liquid matrix capable of solidifying so that the upper surface of the epidermis is not covered, which matrix is itself contained in a cell culture insert, the bottom of which consists of a porous membrane,
(d) solidifying the matrix so as to trap the immersed part of the skin explant, where the upper surface of the epidermis is not covered, and to cause the solidified matrix to adhere to the side walls and the porous membrane of the insert,
(e) putting the culture insert containing the skin explant obtained in step (d) in a culture chamber containing appropriate culture medium, and
(f) culturing the skin explant.

By *"ex-vivo* skin explant" is meant a skin fragment that comprises at least the epidermis, dermis, and epidermal appendages. This skin fragment or biopsy may also comprise a portion of the subcutaneous tissue also called the hypodermis. The hypodermis is located immediately below the dermis and forms a protective cushion separating the skin from the fibrous membranes surrounding the deeper organs, muscles, tendons, and bones. The hypodermis consists of adipocytes, nerves, a network of blood and lymphatic capillaries, a small number of fibroblasts involved in the synthesis of extracellular matrix components, and immune cells such as dendritic cells and macrophages. The hypodermis is divided into adipose lobules containing adipocytes and separated by connective walls that allow the passage of nerves and vessels. The functions of the hypodermis are to isolate, to provide a reserve of energy to the skin cells, and to absorb physical stress. In addition, studies have shown the important role played by lymphatic transport in the absorption of peptide-based drugs.

Preferably, the *ex-vivo* skin explant has a thickness ranging from 1 millimeter (mm) to 1 centimeter (cm), more particularly from 2 mm to 6 mm, even more particularly from 2 mm to 4 mm. A thickness of up to 1 cm is obtained for *ex-vivo* skin explant comprising the epidermis, dermis, epidermal appendages, and hypodermis. In all cases, the thickness of the *ex-vivo* skin explant is not less than 1 mm.

In a particular embodiment, the *ex-vivo* skin explant comprises the epidermis, dermis, epidermal appendages, and between 5 and 15 mm of hypodermis and, preferably, between 5 and 10 mm of hypodermis.

In another particular embodiment, the hypodermis is removed from the *ex-vivo* skin explant prior to inserting the implantable microfluidic device.

Preferably said *ex-vivo* skin explant is taken from an animal, in particular a mammal, and preferably from a human. As biopsies that can be used in the method of the invention may be mentioned those obtained from surgical waste or slaughterhouses. By "surgical waste" is meant skin samples obtained from cosmetic surgery, including post-blepharoplasty, lift, abdominoplasty, cruroplasty, brachioplasty or breast reduction. Skin biopsy sampling techniques are well known to the one skilled in the art.

In order to ensure its *in-vitro* survival, sampling of the *ex-vivo* skin explant must have been carried out within 1h to less than 72h, preferably within 1h to less than 48h, before inserting an implantable microfluidic device in it.

The *ex-vivo* skin explant may be issued from healthy skin biopsy. By "healthy skin biopsy" is meant a skin fragment that does not show any sign of inflammation perceptible to the eye (namely break in the skin, redness, swelling, heat, or excessive scaling...), or express any inflammation marker. Furthermore, it is imperative that the healthy skin biopsy used in the method of the invention be taken from an individual with no dermatological pathology, in particular inflammatory.

It is also possible to treat a healthy skin biopsy using UV light to create an erythema. Such biopsies allow the study of compounds capable of reducing this type of skin lesion.

The *ex-vivo* skin explant may be issued from diseased skin biopsy. By "diseased skin biopsy" is meant a skin fragment that shows particular damage related to skin pathologies such as for example atopic dermatitis, psoriasis, eczema, dermatoses, allergic contact dermatitis, lichen, pruritus, Netherton's syndrome, ichthyosis vulgaris ...

Preferably, the *ex-vivo* skin explant has a cylindrical shape.

However, any other geometrical shape of *ex-vivo* skin explant is also suitable for the method according to the invention, in particular a shape which is square, rectangular, oval, triangular, etc.

Preferably, the cylindrical shape has a diameter ranging from 1 mm to 50 mm, more particularly from 5 mm to 20 mm, even more particularly from 7 mm to 17 mm, and a thickness varying from 1 mm to 20 mm, more particularly from 2 mm to 15 mm, even more particularly from 2 mm to 10 mm, and even more particularly from 2 mm to 5 mm.

Preferably, the surface of the *ex-vivo* skin explant is about 1 cm² to about 10 cm².

By "implantable microfluidic device" is meant a thin, rigid, flexible or semi-flexible hollow tube. By "flexible" means capable of being bent or flexed at least under the effect of a lateral action due to the displacement of a fluid. A deformable or stretchable implantable microfluidic device may recapitulate most of the physiological properties of blood vessels, i.e. lower inner hydrodynamic resistance, wall porosity and deformability. The implantable microfluidic device is biocompatible, and may not trigger physiological events. The material of the implantable microfluidic device may not cause inflammation, immune response, infection, or any other sort of rejection within the *ex-vivo* skin explant. It could be functionalized with biologically active species or cells. Examples of a suitable material include, but are not limited to, silicone rubber, nylon, polyurethane, polyethylene terephthalate (PET), latex, Teflon, hydrogel materials and thermoplastic elastomers. In all embodiments, the two extremities of the implantable microfluidic device are open, thus enabling the transport of solutions, suspensions or compositions.

Preferably, the external diameter of the implantable microfluidic device is from 0.3 mm to 2.5 mm, as an example from 0.7 mm to 2.2 mm, more preferably from 0.9 mm to 1.3 mm, and most preferably is 1.1 mm.

Preferably, the internal diameter of the implantable microfluidic device is from 0.05 mm to 2.25 mm, as an example from 0.51 mm to 1.75 mm, more preferably from 0.61 mm to 0.96 mm, and most preferably is 0.9 mm. With an internal diameter superior to 100 µm, the inner hydrodynamic resistance is lowered.

Preferably, the length of the implantable microfluidic device is from 19 mm to 50 mm, more preferably from 25 mm to 45 mm, and most preferably is 32 mm.

In all embodiments, the total length of the implantable microfluidic device is greater than the size of the *ex-vivo* skin explant. For example, if the *ex-vivo* skin explant is cylindrical in shape, the total length of the implantable microfluidic device is greater than the diameter of the *ex-vivo* skin explant.

The implantable microfluidic device may be a catheter, a microdialysis probe or any material suitable for being implanted in an *ex-vivo* skin explant. Preferably, the implantable microfluidic device is a catheter.

In order to allow the administration, the delivery, the diffusion of the species from an injected solution towards the *ex-vivo* skin explant or to provide an efficient and continuous collection of the metabolic wastes or molecules secreted by the *ex-vivo* skin explant, the portion of the implantable microfluidic device that passes through the ex-*vivo* skin explant is porous.

Preferably, in the method of the invention, the portion of the implantable microfluidic device that passes through the *ex-vivo* skin explant is porous.

In all embodiments, the porosity is ensured by a plurality of pores, i.e. micro holes or apertures, arranged along the length of the portion of the implantable microfluidic device that passes through the *ex-vivo* skin explant. The pores can be arranged on all sides of the implantable microfluidic device to ensure a homogeneous diffusion around it, or only on certain sides to direct the diffusion. Pores may be formed using well established standard techniques such as 3D lithography, i.e. photolithography or etching, water-jet cutting, micro-mechanical drilling or laser drilling.

The rate of infusion and diffusion from the porous portion of the implantable microfluidic device into the *ex-vivo* skin explant can be influenced by the pore size, shape and their number. The average pore size, or pore diameter, can be at least about 10 µm to about 250 µm, more preferably from about 20 µm to about 100 µm, most preferably of about 50 µm. All the pores can have the same pore size or different pore sizes. The pore sizes can vary by as much as 5%, 10%, 20% or even 30% when all pores have the same pore size. The pore size can vary by as much as 50%, 100%, 200%, 300%, 400% or more when pores have different pore sizes. The pore size can be adapted to provide a desired diffusion rate for a specific species or compound.

Preferably, in the method of the invention, the porous portion of the implantable microfluidic device is obtained by making pores having a diameter from about 40 µm to about 250 µm, more preferably from about 50 µm to about 200 µm, most preferably of about 50 µm.

The pore size is a relevant criterion for the selection of molecules that will be able to enter or leave the implantable microfluidic device. In fact, the pore size also acts as a molecular weight cut-off.

The pore shape may also be a relevant criterion for the selection of molecules that will be able to enter or leave the implantable microfluidic device. Thus, the pores can be round, square, rectangular, triangular, star-shaped, loophole-shaped, ... When pores are rectangular or loophole-shaped, they are about 50 µm wide and over 100 µm long.

The rate of infusion and diffusion from the porous portion of the implantable microfluidic device into the *ex-vivo* skin explant can be influenced by the pressure drop across the implantable device. This pressure drop results from the difference between the inlet pressure of the solution injected into the implantable device and its outlet pressure. The atmospheric pressure is the reference point above the epidermis of the skin explant.

To facilitate the inserting of the implantable microfluidic device into the *ex-vivo* skin explant, one of the two extremities of the device may be sharpened. This sharpened end limits the risk of rupture, tearing of the skin explant at the time of insertion of the implantable device.

The implantable microfluidic device can be inserted in the dermis or the hypodermis of the *ex-vivo* skin explant. The choice of the skin layer for receiving the implantable device depends on the intended application.

Preferably, in the method of the invention, the implantable microfluidic device is inserted into the dermis. In order to avoid tearing the *ex-vivo* skin explant, the insertion of the implantable microfluidic device should take place just below or in close proximity to the epidermis.

By "close proximity to the epidermis" means that the implantable microfluidic device is inserted between 0.1 mm and 3 mm, preferably between 0.25 mm to 1 mm, below the epidermis.

In all embodiments, the implantable device is inserted substantially parallel to the dermis or the hypodermis of the *ex-vivo* skin explant. By "substantially parallel to the epidermis" is meant that the longitudinal axis of the implantable microfluidic device is substantially parallel to the epidermis layer of the *ex-vivo* skin explant. By "substantially parallel" is intended the same as or very close to parallel. A slight deviation from strict parallelism necessitated by the physical separation is acceptable. As an example, a deviation of less than 10° is acceptable, preferably from less than 5°.

By "protruding slightly" means that both ends of the implantable microfluidic device emerge from the *ex-vivo* skin explant so as to position the porous portion of the microfluidic device in the center of the skin explant. Moreover, both ends of the implantable microfluidic device has to be free to be connected with the other connecting elements of the culture device. The length of the protruding ends may be up to 10%, 20%, 30%, 40%, 50% or 60% of the total length of the implantable device. In all embodiments, it is essential that the length of the implantable microfluidic device is superior to the length of the *ex-vivo skin* explant so as to cross the ex-vivo skin explant from side to side.

Preferably, in the method of the invention, the length of the two ends of the implantable microfluidic device protrude from the *ex-vivo* skin explant by a length of at least 10%, more preferably at least 30%, and more preferably 50% of the total length of the microfluidic implantable device.

As an example, for an *ex-vivo* skin explant having a diameter of 15 mm and an implantable microfluidic device having a length of 32 mm, the length of each of the protruding ends is about 8.5 mm. In this example, the portion of the microfluidic implantable device that passes through the ex-vivo skin explant has a length of about 15 mm.

The microfluidic implantable device can be inserted in the *ex-vivo* skin explant manually, using a curve clamp or a device comprising two stackable parts, one of which has a notch that is adapted to guide the implantable device during the step a) of the method according to the invention.

In step (b) of the method of the invention, the implantable microfluidic device that crosses the *ex-vivo* skin explant is connected to two separate tubings. The tubings have diameter that are similar or different. In all embodiments the diameter of the tubing is suitable to fit over the ends of the implantable microfluidic device. Each ends of the microfluidic implantable device is connected to a tubing, directly or using, for example a male or female luer lock connector. This type of connector is illustrative and should not restrict the scope of the present invention. The material of tubing is biocompatible, flexible or rigid, has low kink ability, torque strength, and lubricity. When the implantable microfluidic device is a catheter, one of the tubing is connected to a hub present at one end of the device.

By "liquid matrix capable of solidifying", also named solidifying or solidifiable matrix, is meant any liquid solution comprising at least one specific compound or composition the concentration of which in said liquid solution is such that, when implementing suitable conditions, especially particular temperature conditions, the liquid solution takes on a solid or gel-like consistency. Now, the nature of this solidifiable matrix must allow the *ex-vivo* skin explant cells to remain alive, i.e. the matrix has no cytotoxic effect and has a solidification/polymerization temperature at room temperature (i.e. from 15°C to 25°C). Said specific compound or composition may be of animal, vegetable or synthetic origin, or a mixture thereof, its nature and its concentration are determined according to the desired physico-chemical characteristics of the matrix when solidified, especially the flexibility and strength of the matrix. The volume of the liquid matrix will be 1/3 to 2/3 of the total volume of the cell culture insert, preferably 2/5 to 3/5 of the total volume; half of the total volume of the insert being the preferred volume.

According to an embodiment of the method according to the invention, in step (c), said liquid matrix capable of solidifying is selected among any liquid solution, preferably nutritive, capable of solidifying or gelling under particular conditions compatible with the survival and the culture of skin cells constituting said *ex-vivo* skin explant, preferably selected from the group consisting of blood plasma or a solution derived from blood plasma (i.e. blood plasma diluted with a physiological buffer to at least 10%, at least 20%, at least 30% and preferably at least 40% w/w of the total weight of the matrix), a fibrinogen solution, a collagen solution, gelatin, synthetic polymeric gels, natural gels, such as agarose gels, in particular agarose or agar-agar gels with low melting points, starch or polysaccharide gels, commercially available matrix such as Matrigel^{®}, or a mixture thereof.

In a more preferred embodiment, the liquid matrix capable of solidifying is composed of two solutions which are mixed together in the culture insert prior to step (c) of immersing the *ex-vivo* skin explant. This matrix is then composed of a first solution selected from a blood plasma solution, a solution derived from blood plasma, a fibrinogen solution, a collagen solution, and mixtures thereof, and a second solution of agar-agar or low melting point agarose.

The first solution, selected from a blood plasma solution, a blood plasma-derived solution, a fibrinogen solution, a collagen solution, or mixtures thereof, is advantageously a nutrient solution. Now, this first solution is mainly able to solidify under the action of an increase or decrease in temperature and/or by the addition of a specific compound or composition. Preferably, the compound allowing this first solution to solidify is the Ca²⁺ ion. Thus, the liquid matrix has a Ca²⁺ concentration of between 1 mM and 5 mM, preferably between 1.5 mM and 4.5 mM; which concentration will cause it to solidify.

According to a first particular embodiment, the liquid matrix has a Ca²⁺ concentration of between 1 mM and 2 mM, preferably between 1.2 mM and 1.4 mM.

According to a second particular embodiment, the liquid matrix has a Ca²⁺ concentration comprising 2 mM and 3 mM, preferably between 2.5 mM and 2.9 mM and more preferably 2.8 mM of Ca²⁺.

It should be noted that in the case of a blood plasma solution or a solution derived from blood plasma, the solution is first treated with an anticoagulant agent with reversible properties. To do so, this solution comprises at least one anti-fibrinolytic agent, such as sodium citrate, tranexamic acid or aprotinin, and in sufficient concentration to obtain the desired anti-fibrinolytic activity. Preferably, the liquid matrix has a final concentration (weight/total matrix weight) of between 2 and 5% of this at least one anti-fibrinolytic agent.

Now, the first solution will preferably be a fibrinogen solution.

The second solution of low melting agar-agar or low melting agarose is preheated for a time and at a temperature sufficient to be liquid and to remain liquid at about 37°C for the time sufficient to be mixed with the first solution in said insert and until the time of immersion of the skin explant. Typically, this second solution is preliminarily heated to its melting temperature or to a slightly higher temperature, preferably to a temperature between 65°C and 70°C. The choice of agar-agar or low melting point agarose is made so as to benefit, for a 1.5% solution (weight/weight total composition), from a gelling temperature between 24°C and 28°C, and a melting temperature higher than 65.5°C. As an example, we can mention the agarose called LMP Agarose Low melting point (GIBCOBRL, LIFE TECHNOLOGIES). Still in connection with this second solution, its concentration of agar-agar or low melting point agarose is between 1% and 5% (preferably in a physiological solution), more preferably between 2% and 5%, between 3% and 4.5%, between 3.5% and 4.5% or between 3.8% and 4.2% or between 3.9% and 4.1%, 4% being the most preferred concentration (by weight in relation to the total weight of the composition). At this concentration and once heated to its melting temperature or to a slightly higher temperature, this second solution of agar-agar or low melting point agarose can be kept in liquid form for at least 1 hour at 37° or, ideally, at least 4 hours, 10 hours or 16 hours. Preferably, the solidifiable liquid matrix comprising said first and said second solution of agar-agar or low-melting agarose has a final concentration of agar-agar or low-melting agarose of between 0.1% and 2%, preferably between 0.2% and 1.8% (weight/weight total matrix). Such a concentration makes it possible not only to obtain a matrix which, once solidified, makes it possible to retain the three-dimensional structure and to keep said fragment or *ex-vivo* skin explant alive, but also to obtain a matrix which is solid but sufficiently flexible to be non-brittle and resistant to point shocks. The solidification of this liquid matrix taking place after deposition of the *ex-vivo* skin explant, leaving the device thus obtained at a temperature of between 37°C and room temperature, preferably at 20°C.

According to a first particular embodiment, the final concentration of agar-agar or low melting point agarose in the liquid matrix (comprising the first and said second solution) is between 0.5% and 2%, preferably between 0.5% and 1.25%, more preferably between 0.5% and 1.0%, with a concentration of 0.7% (weight/weight total matrix) being the most preferred concentration.

According to a second particular embodiment, the final concentration of agar-agar or low melting point agarose in the liquid matrix (comprising the first and said second solution) is between 0.1% and 2%, preferably between 0.2% and 1.75%, 0.25% being the most preferred concentration. Such a concentration makes it possible to obtain a matrix which, once solidified, makes it possible both to preserve the three-dimensional structure and to keep the skin explant alive, and simultaneously to obtain a matrix which is sufficiently flexible to be non-brittle and resistant to mechanical effects applied to the skin explant. The solidification of this liquid matrix takes place after immersion of the skin explant by allowing the assembly to cool down.

According to another preferred embodiment, the liquid matrix capable of solidifying further comprises cells other than the cells that make up the skin explant, which cells are selected from the group consisting of fibroblasts, endothelial cells and nerve cells. Preferably these cells are fibroblasts and ideally primary fibroblasts (as opposed to fibroblast cell lines), such as dermal fibroblasts obtained from human foreskin. These primary fibroblasts, especially dermal fibroblasts, can be prepared and obtained from standard methods well known to the skilled person (see for example HOWARD BV et al, A new method for the establishment of diploid fibroblast cell cultures from human foreskins, Proc. Soc. Exp. Biol. Med. vol.153(2), p:280-3, 1976). Preferably, these cells, and in particular fibroblasts, are contained in the matrix at a concentration between 5.10³ and 5.10⁵ cells/ml, preferably between 10⁴ and 10⁵ cells/ml, the range between 3.10⁴ and 5.10⁴ cells/ml being the most preferred concentration range.

In addition, the liquid matrix may include various compounds such as preservatives, pH agents, etc. For example, the liquid matrix will contain between 5 and 500 mg/mL of ascorbic acid, preferably between 25 and 75 mg/mL, with a preferred ascorbic acid concentration of 50 mg/mL.

According to a third, also preferred embodiment, the liquid matrix capable of solidifying is a solution derived from blood plasma and comprises:
a) 25 to 75% (total volume/volume) fibrinogen, preferably 35 to 45% (v/v),
(b) 5 % to 12 % (total volume/volume) of a 1 %, preferably 8 %, CaCl₂ salt solution,
c) from 5% to 2%, preferably the anti-fibrinolytic agent being selected from tranexamic acid or aprotinin,
(d) from 0.5% to 4% low melting point agarose, preferably from 1% to 2%, and
e) a physiological solution such as a 0.9% NaCl solution, qsp at 100%.

Matrices capable of solidifying and methods for placing ex-vivo skin explant therein are detailed in European patent EP 2 882 290 B1 and are incorporated herein by reference.

The purpose of the matrix capable of solidifying is to allow the implanted *ex-vivo* skin explant obtained at the end of step (e) of the method of the invention to be cultured, and easier handling thereof. By "cultured" is meant the maintenance of the physiological and morphological state of the *ex-vivo* skin explant, that is to say of all the tissues and cells constituting the biopsy.

The purpose of the matrix capable of solidifying is also to allow the *ex-vivo* skin explant to be long term preserved and/or transported, while allowing stable cell functions within a controlled microenvironment.

According to a particular embodiment, the *in vitro* method according to the invention comprises, prior to step (a), a step of fixing, to the epidermal surface of the implanted *ex-vivo* skin explant obtained at the end of step (a), a ring, consisting of a hydrophobic material, the outer diameter of said ring being similar to the diameter of the epidermal surface of the *ex-vivo* skin explant.

Preferably, the hydrophobic material of the ring is a material that is not toxic to the skin. It may be a paraffin polymer, such as a Parafilm^{®} (SIGMA), or a silicon polymer. According to a particular mode, the ring is prepared from a film of hydrophobic material, by perforating said film according to the desired dimensions. Hence, the ring corresponds to a disc perforated at its center. The thickness of the ring is preferably between 0.1 mm and 2 mm, preferably between 0.1 and 1 mm, more preferably between 0.1 and 0.5 mm, and even more preferably between 0.12 and 0.2 mm.

Said ring may be made of an opaque or translucent material. According to a particular embodiment, the ring is made of an opaque material.

According to a more particular embodiment, said ring is attached to the epidermal surface of the biopsy using glue, preferably added to the lower surface of the ring. Said glue can be selected from any type of material that is not toxic to the skin and which has the effect of adhering the ring to the skin, where this material may be silicon. Preferably, said glue is hydrophobic.

The materials, glues and attachment methods of this hydrophobic ring are detailed in European patent EP 2 882 290 B1 and are incorporated herein by reference.

The cell culture insert is configured to receive the *ex-vivo* skin explant inserted by the implantable microfluidic device. For instance, the structure of commercially available cell culture wells can be modified to create notches to accommodate the protruding ends of the implanted device. The culture insert can be of any shape and can be suspended on piles or using lugs. The bottom of the culture insert consists of a porous membrane with a porosity for preventing the liquid matrix from flowing through the membrane before solidification. Preferably, the porosity of this membrane may be in a range of 0.2 µm to 10 µm, more preferably between 0.4 µm and 8 µm, 8 µm being the preferred porosity. The material of this porous membrane can be polyethylene terephthalate (PET), nitrocellulose, or polycarbonate. Among cell culture inserts may be mentioned those supplied in particular by the NUNC company (Roskilde, Danemark), BD Falcon (BECTON DICKINSON France SAS, 38 801 Le Pont-De-Claix, France), Millicell^{®} (EMD MILLIPORE CORPORATION, Billerica, Mass., USA), or Costar^{®} (Grosseron SAS, 44819 Saint-Herblain France), for example the inserts with a membrane made of polycarbonate, PET or nitrocellulose pre-packaged in multi-well plates with 6, 8, 12, and 24 wells, the membrane porosity of which may vary between 0.4 µm and 8 µm. Typically, the culture insert used in the method of the invention has a PET membrane which porosity ranges from 0.8 µm to 8 µm and is configured for the culture chamber used in the method of the invention.

In step (e) of the method of the invention, the culture chamber is also configured to receive the cell culture insert containing the skin explant obtained in step d). A "culture chamber" is generally defined by a base and a wall. In the present invention, the shape of the wall is suitable for receiving the culture insert containing the *ex-vivo* skin explant implanted by the microfluidic device. The culture chamber may be constructed from a material that withstands sterilization, including, for example, sterilization by irradiation (beta or gamma radiation), steam autoclave, ethylene oxide, chemical disinfectants, or dry heat sterilization. In some embodiments, the culture chamber may be made from a thermoplastic material and/or from a material that is formed, for instance, by injection molding. Examples of materials that are suitable for use in the present context include for example, but are not limited to, polyethylene, polypropylene, polystyrene, polycarbonate, polyurethane, polysulfone, polymethylpentene, polydimethylsiloxane (PDMS), polymethylmetacrylate, polyethyleneterepthtalate, polytetrafluoroethylene, or ABS (acrylonitrilbutadiene styrene). However, the examples given here only exemplary in nature a person skilled in the art would readily appreciate how to select other materials suitable for use in constructing the device.

In a particular embodiment, the material of the chamber culture is polydimethylsiloxane (PDMS).

In all embodiments, the bottom of the culture chamber has a rim adapted to receive the cell culture insert containing the *ex-vivo* skin explant inserted by the implantable microfluidic device. The culture chamber holds the cell culture insert and facilitates its handling and transport. In a preferred embodiment, the bottom of the culture insert is at a distance of between 1 and 2.5 mm from the bottom of the culture plate containing it.

In some embodiments, the culture chamber is coated with an agent. By way of example, and without this being restrictive, the coating agent can be selected among bovine serum albumin (BSA), surfactant such as Pluronic^{®} F-127, synthetic, hydrophilic and biocompatible polymer such as Poly(ethylene glycol) (PEG) or, PLL-g-PEG (Poly(L-Lysine)-g-Polyethylene glycol), any other biocompatible natural or synthetic coating molecule, or a mixture thereof. In some other embodiments, the surface of the culture chamber can also be treated with oxygen plasma or PECVD (Plasma enhanced chemical vapor deposition) and thereafter treated with silicon analogue of methane, such as fluorinated or carboxylated silane.

At the end of step (e) of the method of the invention, the *ex-vivo* skin explant has the trade name FlowSkin^{®}.

By "appropriate culture medium" is meant a culture medium containing all the elements necessary for the survival of the skin explant such as for example William's E, DMEM, KBM, ... This culture medium, by its nature as much as by its volume in the culture chamber, helps the survival of the *ex-vivo* skin explant and the cells constituting it over time. In addition to the survival of the cells of the *ex-vivo* skin explant, the culture medium makes it possible, in particular by limiting the stress on them, to maintain the cells of the *ex-vivo* skin explant in their initial state, whether in structural or functional terms.

The presence of culture medium in the culture chamber allows the step (f) of culturing the *ex-vivo* skin explant for several days. By "culturing" is meant maintaining physiological and morphological state of the *ex-vivo* skin explant, that is to say of all the tissues and cells constituting the explant. Thus, the aim of this step is to limit the phenomena of cell death and to maintain the state of differentiation of the cells (in fact to limit the phenomena of dedifferentiation or inappropriate differentiation).

Preferably, step (f) of the method of the invention can last at least 5 days, 6 days, 7 days, 8 days, 9 days, 10 days or more.

A cover, a lid or a protective film may be applied on the top of the culture chamber in which the culture insert containing the implanted skin explant of a microfluidic device has been placed in order to seal the culture plate. In this way, the resulting culture chamber can be transported without difficulty by land, sea or air. Indeed, the skin explant is not only firmly held by the solid matrix but also nourished.

The culture chamber can also be equipped with two needles so as to permit further connecting with a complete circulation system. In these embodiments, the two needles pass through two opposite walls of the culture chamber, the needles extending substantially parallel to the bottom of the culture chamber. Each needle has two ends, the first one being located inside the culture chamber; the second end being located outside the culture chamber. The two needles are connected at one of their ends to the tubings, the other one being free for further connecting with a complete circulation system.

In a preferred embodiment, the method of the invention further comprises a step (g) of connecting the tubings to a circulating system comprising means for providing a continuous or discontinuous/pulsatile flow and/or for modulating the difference of pressure between the inlet and the outlet of the implantable device, said connecting being mediated by input and output ports at the inlet and outlet of the implantable device. By controlling the difference of pressure across the implantable device, it enables injection, suction and pressure control within the implantable device in comparison to external pressure (usually atmospheric pressure). When the internal pressure in the implantable device is lower than the external pressure, no leaks can occur. In this embodiment, the tubings act as connectors between the microfluidic implantable device and the needles.

The two needles constitute input and output ports of the culture system obtained in step (g) of the method of the invention. In some embodiments, the inlet port, the culture chamber and the outlet port are configured to provide a continuous or discontinuous flow of fluid through the *ex-vivo* skin explant. For this purpose, the culture system obtained in step (g) of the method of the invention, includes or is coupled to, means for achieving a flow of fluid in the implantable microfluidic device. These means include any system capable of imposing a pressure drop by controlling the level of the liquid, i.e. the hydrostatic pressure. This passive method, heavily used in biology, is quite similar to the infusion system used clinically *in-vivo.* The circulating system may also include a pump, such as for example a syringe pump, a peristaltic pump, a pressure regulating pump or a similar device capable of providing a continuous or discontinuous/pulsatile flow having any desired flow rate, or able to modulate the pressure within the implantable device by controlling independently the value of the pressure at the inlet and outlet of the device, thanks to the addition of pressure regulators connected to the inlet and outlet or the integration of one way or multiple ways valves in the microfluidic system, to temporarily blocked the outlet of the system and switch from one reservoir to another. It may be desired to select a flow rate or a pressure that leaves the *ex-vivo* skin explant intact or that does not substantially, not significantly, or not at all interfere with the integrity, the biological function or the physiological characteristics of the skin explant. In some embodiments, the flow has a flow rate that is substantially equivalent to an *in vivo* hemodynamic flow rate and/or has a pressure that is substantially equivalent to an *in vivo* hemodynamic pressure. The perfusion should recapitulate a native skin function.

Preferably, the flow rate of a fluid perfused within the *ex-vivo* skin explant ranges from about 0 µl/minute to 1000 µl/minute, as for example about 1 µl/minute to 20 µl/minute, more preferably from about 2.5 µl/minute to 10 µl/minute, and most preferably from about 2.5 µl/minute to 5 µl/minute. In a more preferred embodiment, the flow rate of the fluid perfused through the *ex-vivo* skin explant using the implantable microfluidic device is 2.5 µl/minute.

In another embodiment, pressure within the implantable device can be controlled with pressure differences between inlet and outlet ports. By exercising pressure-vacuum cycles, the flow of a fluid perfused within the *ex-vivo* skin explant is regulated and the internal pressure inside the implantable device remains below of above the pressure of caused by the air on the epidermis.

Preferably, the difference of pressure accross the implantable device ranges from about -1 bar to 1 bar, preferably from about -200mBar to 200 mbar and even more preferably from about -50 mbar to 50 mbar.

Cycle of pressure may also be modulated from about 1.10⁻³ to 100 Hertz.

Injection and pressure within the implantable device can be continuous, discontinuous or transient.

In one embodiment, the implantable microfluidic device is used to deliver at least one compound of interest through the *ex-vivo* skin explant. Such a compound of interest can be culture media, oxygen carrier or a drug.

By "oxygen carrier" is mean any composition or compound that allows a physiological oxygen supply to the cells in the *ex-vivo* skin explant. Oxygen is delivered according to their needs. More interesting are oxygen carriers that also have intrinsic antioxidant activity so as to protect the cells from the harmful effect of free radicals generated by cellular respiration. Perfluorocarbon- and hemoglobin-based substitutes are oxygen carrier suitable for the purpose of the present invention.

By "drug" is mean any agent or compound that modulate the metabolic activity of the cells or which has a physiological effect on the *ex-vivo* skin explant. Therapeutic compounds are of particular interest. Among them, one can cite small molecules, peptides, proteins such as for example monoclonal antibodies, nucleic acids, or any other compounds that can be used topically of in a systemic way to cure or alleviate a pathological condition.

In one particular embodiment, the method of the invention is for further detecting and/or quantifying at least one biomarker of interest contained in the liquid secreted by the *ex-vivo* skin explant, and said method further comprises the following steps:
(h) collecting the liquid secreted by the *ex-vivo* skin explant through the output port, and
(i) isolating, identifying, and optionally quantifying the at least one biomarker of interest.

By "biomarker of interest" is meant any compound, analyte or molecule implied in metabolic activities of cells of the *ex-vivo* skin explant. For example, the concentration of known markers of metabolic stress and fatigue (i.e. lactate, glucose, ketones, cortisol, and TNF-α) in extracted liquid secreted by the *ex-vivo* skin explant can be determined using standard assays. These assays require between 1 and 50 microliters of sample fluid. A NANODROP^{®} ND100 spectrophotometer capable of analyzing 2 µl volumes of solution can be used if the extracted IF volumes are insufficient for standard assays. The IF biomarker concentrations can be correlated with levels found in whole blood or serum. The Human Metabolome Database (HMDB, www.hmdb.ca) and the literature searches can be used to identify useful biomarkers. These markers can be changed according to the need. Mass spectrometry, HPLC, infrared can be used to directly analyze the biomarker composition of extracted IF.

The liquid secreted by the *ex-vivo* skin explant can be compared to interstitial fluid existing in in-vivo skin. By "interstitial fluid' (IF) is meant a fluid that surrounds cells and tissues throughout the body and is formed by extravasation of plasma from capillaries and modified by metabolic and other processes in the tissue. Interstitial fluid shuttles nutrients and waste products between blood vessels and cells and is roughly a combination of serum and cellular materials. Even though 83% of proteins found in serum are also in IF, about 50% of proteins in IF are not present in serum, suggesting that IF may be a source of unique biomarkers. Moreover, IF is also interesting for continuous monitoring due to absence of clotting factors. Skin is the most accessible organ and therefore an attractive source of IF containing both systemic and dermatological biomarkers. Suction using a pump to create negative pressure on the skin surface, or osmotic driving force can be used to flow IF from skin through the implanted microfluidic device to a sample reservoir. For example, a pump can be a vacuum pump, a capillary force pump, a microdialysis pump, or a pulsatile vacuum pump. Saline solution can also be injected through the implantable microfluidic device and spread in the *ex-vivo* skin explant by the pores of the porous portion of the implantable microfluidic device.

In one preferred embodiment, the step (h) of collecting the liquid secreted by the ex-*vivo* skin explant is done repeatedly at regular or irregular intervals so as to follow up the concentration of said at least one biomarker of interest.

The success of topical and transdermal administration of drugs is directly related to the methods used for evaluation of the formulations, which enable optimization of the skin absorption of the drug so that it can reach effective drug concentrations at the therapeutic site. Cutaneous microdialysis as implemented in the present invention is a technique in which a microfluidic device is implanted in the dermis or hypodermis of the *ex-vivo* skin explant, directly under the formulation to be tested.

In another embodiment, the method of the invention is for further assessing permeation of the at least one biomarker of interest through the skin, said method further comprises a step (g') of applying topicallyto the epidermis of the *ex-vivo* skin explant a composition comprising the at least one biomarker of interest after the step (g) of connecting the needles with the tubings and prior to the step (h) of collecting the liquid secreted by the *ex-vivo* skin explant.

By "permeation" is meant the diffusion of a compound into a certain skin layer. Permeation should not be confused with skin absorption that implies that the compound becomes systemically available, nor with skin penetration which is the diffusion into deeper layers. Permeation is diffusion of a penetrant into a certain skin layer. Subsequent diffusion through that layer represents penetration. Penetration through layers of skin to either the site of action or systemic circulation represents absorption. Absorption through the skin considers full penetration of the skin layers so that the penetrant can become systemically available.

In this particular embodiment, the at least one biomarker of interest can be a cosmetic agent, a gas, a potentially dangerous exogenous agent from the environment, etc...

In this preferred embodiment, the step (h) of collecting the liquid secreted by the ex-*vivo* skin explant is done repeatedly at regular or irregular intervals so as to assess permeation rate of said at least one biomarker of interest.

In another embodiment, the method of the invention is for further assessing the activity of at least one compounds intradermally injected, said method further comprises a step (g") of injecting a composition comprising the at least one compound of interest after the step (g) of connecting the needles with the tubings and prior to the step (h) of collecting the liquid secreted by the *ex-vivo* skin explant, the composition being injected in the dermis of the *ex-vivo* skin explant by using the microfluidic implantable device.

In this embodiment, the purpose is to identify macro- and micro-signs of modification or alteration of the epidermis, dermis or hypodermis layer. By "signs" is meant any perceptible change such as, but with no limitation to, dryness, cracking, peeling, redness, redness, thickening, thinning, or any other sign occurring in the epidermis.

In another embodiment, the method of the invention is for further assessing the activity of at least one compounds subcutaneously injected, said method further comprises a step (g'") of injecting a composition comprising the at least one compound of interest after the step (g) of connecting the needles with the tubings and prior to the step (h) of collecting the liquid secreted by the *ex-vivo* skin explant, the composition being injecting in the hypodermis of the skin explant by using the microfluidic implantable device.

By injecting the composition in the hypodermis, the purpose is to mimic extravasation that naturally occurs *in-vivo* from skin vasculature.

In another embodiment, the method of the invention is for further studying inflammation of the skin, said method further comprises a step (g"") of injecting a composition comprising a cocktail of cytokines, after the step (g) of connecting the needles with the tubings and prior to the step (h) of collecting the liquid secreted by the *ex-vivo* skin explant, the composition being injecting in the dermis or hypodermis of the skin explant by using the microfluidic implantable device.

In one preferred embodiment, the cocktail of cytokines comprises an effective amount of pro-inflammatory cytokines. Preferably, the cocktail of cytokines is a mixture of interleukine-1 beta (IL1β), tissue growth factor beta (TGF-β) and interleukin-23 (IL23). This cocktail enables the differentiation of activated resident T cells into LTh1 and/or LTh17 T helper cell lineages. By "effective amount" is meant an amount that is sufficient to achieve the expected effect. In the instant case, the expected effect is obtaining the polarization of the activated resident T cells into LTh1 and/or LTh17, and the synthesis of inflammation markers.

Preferably, the concentration of IL-1β in the mixture of the composition used in step (g"") of the method of the invention ranges from 1 ng/ml to 50 ng/ml, preferably from 5 ng/ml to 30 ng/ml, and particularly preferably from 7 ng/ml to 15 ng/ml.

Preferably, the concentration of IL-23 in the mixture of the composition used in step (g"") of the method of the invention ranges from 10 ng/ml to 100 ng/ml, preferably from 20 ng/ml to 80 ng/ml, and particularly preferably from 30 ng/ml to 60 ng/ml.

According to a particular embodiment, the concentration of IL-23 in the mixture of the composition used in step b) of the method of the invention is 50 ng/ml.

Preferably, the TGF-β concentration in the mixture of the composition used in step (g"") of the method of the invention ranges from 1 ng/ml to 50 ng/ml, preferably from 5 ng/ml to 30 ng/ml, and particularly preferably from 7 ng/ml to 15 ng/ml.

According to a more particular embodiment, the concentrations of IL-1β, IL-23, and TGF-β in the mixture of the composition used in step (g"") of the method of the invention are 10 ng/ml, 50 ng/ml, and 10 ng/ml, respectively.

Preferably, step (g"") of injecting the ex-vivo skin explant with a composition comprising at least one mixture of IL-1β, IL-23, and TGFβ is carried out for a period of at least 3 days, preferably between 3 and 10 days.

Prior to the step (g"") of injecting the cocktail of cytokines in the *ex-vivo* skin explant as described above, it may be necessary to activate resident T cells in the skin. Such activation can be obtained by injecting a composition comprising an effective amount of an anti-CD3 antibody and an anti-CD28 antibody, and optionally IL-2 into the dermis of the *ex-vivo* skin explant. Activation of resident T cells in the dermis can be highlighted directly using activation markers, such as, but not limited to, CD69. The effective amounts of anti-CD3 antibody, anti-CD28 antibody, and optionally IL-2 vary according to the size of the *ex-vivo* skin explant.

As an example of an effective quantity may be mentioned the use of injection volumes in relation to the concentrations listed above.

According to a particular embodiment, the effective amounts of anti-CD3 antibody, anti-CD28 antibody, and IL-2 in the composition injected into the dermis of the *ex-vivo* skin explant according to the method of the invention are 500 ng, 500 ng, and 0.1 ng, respectively, for a biopsy with a diameter equal to 8 mm.

According to another particular embodiment, the effective amounts of anti-CD3 antibody, anti-CD28 antibody, and IL-2 in the composition injected into the dermis of the *ex-vivo* skin explant according to the method of the invention are 1750 ng, 1750 ng, and 0.35 ng, respectively, for a biopsy with a diameter equal to 15 mm.

The pore size of the implantable microfluidic device allows antibodies to diffuse from the lumen of the device toward the dermis of the *ex-vivo* skin explant. Advantageously, the injection of the composition comprising anti-CD3 and CD28, and optionally IL-2 using the implantable microfluidic device makes it easier to reach the resident T cells in the dermis which are randomly distributed.

Methods for obtaining inflamed ex-vivo skin explant are detailed in international publication WO 2019/063122 A2 and are incorporated herein by reference.

According to a particular embodiment of the invention, the *ex-vivo* model of inflamed skin obtainable by the method described above allows compounds for treating inflammatory skin diseases to be identified. Among inflammatory skin diseases may be mentioned, but with no limitation, atopic dermatitis, psoriasis, eczema, dermatoses, allergic contact dermatitis, lichen, pruritus, Netherton's syndrome, ichthyosis vulgaris...

In a second aspect, the present invention relates to a system obtainable at the end of step (d) of the method of the invention, said system comprising a cell culture insert the bottom of which consists of a porous membrane, said culture insert containing an *ex-vivo* skin explant embedded in a solidified matrix, wherein the *ex-vivo* skin explant comprises the epidermis, the dermis and optionally the hypodermis characterized in that an implantable microfluidic device is inserted through the dermal or hypodermal part of the *ex-vivo* skin explant, wherein the microfluidic device is positioned substantially parallel to the epidermis and traverses the *ex-vivo* skin explant from side to side, both ends of the device protruding slightly beyond the *ex-vivo* skin explant.

In the system of the invention, the portion of the implanted microfluidic device that passes through the skin is porous.

In another embodiment, the system of the invention further comprises a culture chamber configured to receive the cell culture insert containing the ex-vivo skin explant. In a preferred embodiment, the culture chamber further comprises a lid or a protective film.

The material suitable for the culture chamber is as described above.

The ex-vivo skin explant in the system of the invention is preferably a mammalian skin explant, preferably a human skin explant, preferably obtained from surgical specimens.

An advantage of the system of the invention is to provide an accurate control of the fluidic exchange in the skin both at spatial and temporal level. The system of the invention thus offers a consistent delivery of nutrients via a continuous or alternate perfusion through the pores of the implantable microfluidic device to ensure cell survival throughout the entire *ex-vivo* skin explant for optimal structural and physiological maintenance. Moreover, connecting the system of the invention to a flow control system not only provides oxygen and nutrients to the skin explant but also potentially increases waste product removal.

The system of the invention is suitable for several uses, such as to study pharmacokinetic and/or pharmacodynamic profile of drugs. Unlike the traditional pharmacokinetic evaluations assess the total drug in the sample (drug-protein binding and free drug fraction), the system of the invention allows separate calculation of the free and protein-bound fractions in the *ex-vivo* skin explant.

Another advantage offered by the system of the invention is that the free drug fraction only in the liquid secreted by the *ex-vivo* skin explant diffuses into the implanted microfluidic device in the collecting tubes.

Other possible uses for the system of the invention include the following ones:
- to identify percutaneous penetration of potentially dangerous exogenous agents from the environment,
- to administer high molecular weight molecules in a time-controlled manner,
- to study the diffusion of an infused compound into the dermis or hypodermis,
- to compare different routes of administration,
- to test different modes of administration (over time),
- to study skin inflammation,
- to assess toxicity of drugs administered by subcutaneous route,
- to assess bioavailability of a cosmetic agent administered by topical route,
- to assess permeation rate of a compound administered by topical route, or
- to obtain long term culture of *ex-vivo* skin explant.

By "assessing toxicity of drugs administered by subcutaneous route" is intended to mimic the administration of a drug - that occurs in the hypodermis - by the administration in the *ex-vivo* skin explant by using a porous microfluidic implantable device. Potential toxic effects of the drugs can then be assessed by histological analysis.

Long term culture of *ex-vivo* skin explant is of high interest for many *in-vitro* studies such as repeated dose toxicology and development of pathological models.

Inserting of the microfluidic implantable device through the dermis or hypodermis of an *ex-vivo* skin explant may be done manually as described above, or by using a device specially designed to hold the skin explant during step (a) of the method of the invention.

In a third aspect, the present invention relates to a device for inserting a microfluidic implantable device into an *ex-vivo* skin explant, said device comprising two separate and removable stackable parts which, once assembled together, form:
(a) a central cavity suitable for receiving and maintaining an *ex-vivo* skin explant, and
(b) ducts through which a microfluidic implantable device is guided to go through the *ex-vivo* skin explant from side to side.

Preferably, the central cavity of the device is formed by the assembly of a recess existing in the center of each of the removable and stackable parts and the conduits result from the assembly of two grooves present in each of the removable and stackable parts, each of the grooves being in contact with the recess.

In a preferred embodiment, the device further comprises fastening means, such as for example magnets, protrusions, pliers, screws, metal springs, clips, clamps.

In another preferred embodiment, the device of the invention further comprises means to prevent the skin explant from slipping off the device. As an example, spikes or pins may be present at the inner surface of at least one of the stackable parts.

Preferably, one of the removable and stackable parts of the device of the invention is transparent.

In a fourth aspect, the present invention relates to a method for inserting a microfluidic implantable device in an ex-vivo skin explant, comprising the steps of:
(a) placing the skin explant in the recess of the first stackable part of the device as defined previously, where the epidermis is in contact with this first element and the dermis or hypodermis is in contact with the air,
(b) assembling the second stackable part of the mold on the first stackable part containing the skin explant so that the dermis or hypodermis of the explant is in contact with the recess of the second stackable part of the device,
(c) fastening the two stackable parts of the device comprising the skin explant,
(d) introducing the implantable microfluidic device in the groove,
(e) passing the implantable microfluidic device through the skin explant from side to side so as to position the porous part of the implantable microfluidic device is located in the center of the skin explant.

In a preferred embodiment, the method further comprises a step of unlocking the two stackable parts of the device comprising the *ex-vivo* skin explant so as to obtain an *ex-vivo* skin explant inserted with an implantable microfluidic device.

The following examples are provided to illustrate certain preferred embodiments and aspects of the present invention and are not to be construed as limiting the scope thereof.

### EXAMPLES

### I - FlowSkin^{®} model production

### I.1 - Preparation of ex-vivo skin explant

The *ex-vivo* skin explants are prepared from a complete skin biopsy, including the epidermis, dermis, and hypodermis. Based on these biopsies, two different embodiments are possible.

In a first embodiment, the adipose tissue (namely the hypodermis) is cut with curved scissors in order to separate it from the dermis. The biopsies (epidermis and dermis), the thickness of which is about 3 mm, are then cut out using a metal punch. This first embodiment is for testing all types of applications intended by the present invention, except those related to subcutaneous injection.

In a second embodiment, the hypodermis is preserved. The biopsies (epidermis, dermis, and hypodermis), the thickness of which is about 1 cm, are then cut out using a metal punch. This second embodiment is for testing applications related to subcutaneous injection.

Once prepared, the biopsies are maintained floating in buffered saline solution of HBSS supplemented with 1% penicillin/streptomycin and 0.2% amphotericin B until the implantable microfluidic device is inserted.

### I.2 - Microfluidic device preparation

This example illustrates the design of the porous portion of an implantable microfluidic device to be inserted into the dermis or hypodermis of the *ex-vivo* skin explant. Thin-walled siliconized polyurethane catheter Introcan^{®} Certo catheter (B-BRAUN, Sempach, Switzerland) is the implantable microfluidic device selected. The length of the implantable device is 32 mm with internal diameter of the implantable device of 0.9 mm and an external diameter of 1.1 mm. This implantable microfluidic device is suitable for a 15 mm diameter *ex-vivo* skin explant. However, the length and diameters of the implantable microfluidic device can be changed to adapt to varying applications. Figure 3 A is an enlarged schematic representation of the porous portion of the device.

The mechanical design of pores is obtained via laser ablation as shown is figure 3B. Pores having a size of 50 µm (figure 3D) are drilled on the four sides of the porous portion of the implantable microfluidic device for in-plane delivery of fluids, on a length of 10 mm, centered on the device. The distance of the pores of the same side in this example is 0.8 mm, with an offset of 0.2 µm at each 90° rotation. The size, the number and the distance between pores can be changed to adapt to varying applications.

### I.3 - Processing of the culture insert and culture chamber

### I.3.1 - Culture insert

Slots are made in the culture insert to allow the passage of the free ends of the implantable microfludic device and the tubings. These slots are made in the side wall of the culture insert at a sufficient height, and perpendicular to the bottom of the insert (figure 6, A). The slots are sawn up to 3 mm from the bottom of the insert. Under no circumstances should these slots be made in the full height of the side wall of the insert, as their height should not exceed the height of the solidifiable liquid matrix that will be poured into the insert to embed the *ex-vivo* skin explant.

### I.3.2 - Culture chamber

PDMS solution (Sylgard^{®} 184) is obtained by mixing the base and hardener in a 10:1 ratio. Once thoroughly mixed, the solution is degassed in a vacuum bell. This PDMS is then poured between the two parts of a mold which was designed on the FreeCad software. Place the assembly in an oven at 60°C for a minimum of 4 hours, then remove the culture chamber from the mold. 4 small discs of PDMS are glued on the 4 corners of the culture chamber, using a bi-component silicone adhesive. The culture chamber is then wash with 70% ethanol and then autoclave them. A coating is performed using a solution of BSA at 4.5g/L in PBS, and the culture chamber is incubated overnight at 37°C. After washing with PBS, both ends of the culture chamber are pierced with a PDMS punch. Needles are inserted into each hole formed; one corresponds to the inlet of the system, the other to the outlet (figure 5, B).

### I.4 - Implantation of the microfluidic device through the ex-vivo skin explant

The microfluidic device is implanted in the dermis of the *ex-vivo* skin explant, parallel to the epidermis. The implantation should be as close as possible to the epidermis, without piercing it. The microfluidic device is implanted through the *ex-vivo* skin explant. Implantation can be done manually or using an internally developed tool and printed in 3D (Figure 4).

### I.4.1 - Manually

Implantation is performed using curved forceps with the epidermis down and the bevel of the catheter needle upwards. Once implanted, the needle is removed and only the catheter remains in the ex vivo skin explant.

### I.4.2 - With a device

A tool consisting of two stackable and magnetized parts was designed on the FreeCad software and then printed in 3D (figure 4). The *ex-vivo* skin explant is placed on the lower part (A, C and E), epidermis down. The upper part of the tool (B and D) magnetizes on the lower part to block the *ex-vivo* skin explant (F). The catheter is inserted between the two parts in the notch provided for this purpose and then implanted into the *ex-vivo* skin explant until it comes to a stop against the tool (G and H). The two parts are separated to retrieve the *ex-vivo* skin explant. The needle is removed and only the catheter remains in the ex vivo skin explant.

### I.5 -Complete assembly of the ex-vivo skin explant model

As shown in figure 2, a silicone ring (3) with an inner diameter of 12 mm and an outer diameter of 15 mm is glued to a 15 mm diameter *ex-vivo* skin explant (4) surface, using a bi-component silicone glue. The outlet end of the catheter (6) is connected to a microfluidic tubing (8) of about 2 cm long. The inlet end of the catheter is connected to a micro-fluidic tubing (1) of about 2 cm long using a Male Luer-Lock connector (2). A solidifiable liquid matrix is poured in the culture insert (7) placed in the PDMS culture chamber (9), the matrix (5) being a mixture of first solution of fibrinogen and tranexamic acid at a 10:1 ratio and a second solution of agarose 1.07%, 130 mM NaCl and 10 mM CaCl₂. The floating *ex-vivo* skin explant is deposited on the matrix by inserting both ends of the protruding ends of the microfluidic device into the slots of the culture insert provided for this purpose. The figure 1 illustrates a culture insert containing the *ex-vivo* skin explant (1) implanted with a microfluidic device (4) and embedded in the solidified matrix (2). The system is placed at 4°C for a minimum of 10 minutes to allow the matrix to gelify. Lastly, bi-component silicone adhesive is placed all around and over the ring to solidify the model. A lid is placed on the top of the PDMS culture chamber.

### I.6 - Connecting the ex-vivo skin explant model to a perfusion system

Once the adhesive has set, the two tube ends are connected to the needles (10 and 11) placed in the walls of the PDMS culture chamber. The needle corresponding to the inlet is connected to the microfluidic system using a Male Luer-Lock connector and microfluidic tubing. The microfluidic system includes a syringe pump (neMESYS, Cetoni) and a bubble trap (Fluigent). The needle corresponding to the outlet is connected to a micro-fluidic tubing using a Male Luer-Lock type connector. It can be placed into a reservoir to collect the outgoing flow, or connected to a valve in order to modulate pressure inside the catheter by periodically blocking the outlet of the microfluidic system. Figure 5 is a schematic view of the microfluidic system. Culture medium is placed in the well of the culture plate which is then placed in a controlled atmosphere CO₂ incubator (37°C, 5% CO₂). The infusion may be started according to the desired parameters.

### II - Assessment of the impact of infusion on skin physiology

### II.1 - Perfusion of a solution over 10 days

In order to assess the impact of infusion on skin physiology, a comparison between the NativeSkin^{®} and FlowSkin^{®} models' integrity, viability and metabolism has been carried out after 10 days of ex vivo culture. *Ex-vivo* skin explants are issued from several independent donors. The number of skin explant replicates for each condition varies from 2 to 4.

NativeSkin^{®} models are cultured in 2mL of culture medium renewed every day. Culture medium is composed of William'S E (Pan Biotech) commercial medium supplemented with 0.1 % CaCl₂ at 1mM, 1 % Penicillin/Streptomycin, 0.2 % Amphotericin B and 2 % Vitamin C at 10 mg/mL.

FlowSkin^{®} models are cultivated in 2mL of the same culture medium. In addition to the passive diffusion of the medium through the pores of the culture insert, the culture medium is infused at a rate of 5 µl/min for 30 seconds, followed by 0 µl/min for 30 seconds on a cyclic basis for 10 days. No pressure control is performed during this experiment.

### II.1.1 - Skin cells viability and skin integrity

Skin cells viability is assessed using the Cell Counting Kit-8 (Sigma Aldrich). After 10 days of culture, six 2 mm in diameter biopsies were produced from the samples to analyze, and immersed in 250 µL of WST-8 solution diluted at 1X in culture medium. After 3 hours of incubation at 37°C, biopsies were removed and the optical density of the WST-8 solutions was measured using a microplate reader (Victor Nivo, Perkin Elmer) at 450 nm. Optical density of the solution is directly correlated to skin cells viability.

*Ex-vivo* skin explants are issued from 7 donors, numbered 1 to 7 in the x-axis of figure 7. This figure represents the average +/- SEM of the viability measured for each replicates of each condition: in black for the NativeSkin^{®} models and in grey for the FlowSkin^{®} models. Skin viability is expressed as a percentage of fresh skin viability measure the day of models' production.

Figure 7 shows that no significative differences are visible between viability levels of the NativeSkin^{®} (black bars) and FlowSkin^{®} (grey bars) models, with values in the order of 76% of the viability observed at D0.

In order to assess skin integrity, a Hematoxylin and Eosin staining was performed on paraffin skin cross-sections. After 10 days of culture, skin biopsies were cut in half (perpendicularly to catheter implantation axis in the case of the FlowSkin^{®} models) and fixed in 10 % buffered-formalin for 48 hours. Biopsies were then dehydrated in several baths of ethanol and xylene and then embedded in paraffin wax. 5 µm thickness skin cross sections were produced using a microtome. Cross sections were rehydrated in several baths of xylene, ethanol and water, and stained by immersion in Mayer's Hematoxylin for 3 minutes, followed by 2 minutes in Eosin Y. Finally, skin slices were mounted between slides and coverslips using EuKitt (Sigma). Images were acquired using a DMi1 (Leica).

Representative images of NativeSkin^{®} (A) and FlowSkin^{®} (B) models are presented in Figure 8.

Figure 8 shows that after 10 days of infusion, the integrity of the NativeSkin^{®} (A) and FlowSkin^{®} (B) models is preserved, without detectable alteration of the tissue.

### II.1.2 - Skin cells proliferation and apoptosis

Skin cell proliferation and apoptosis is assessed using an anti-Ki67 and an anti-cleaved Caspase-3 immunostaining respectively, performed on 5µm paraffin cross-sections (similar protocol of fixation, dehydration and paraffin embedding than for the Hematoxylin and Eosin staining). After rehydration, skin slices were unmasked for 20 minutes in Target Retrieval Solution, pH9 (Dako) at 95°C in a water bath. They were then blocked and permeabilized for 40 minutes at 56°C with 5% (v/v) goat serum in PBS plus 0.5% Triton X-100 (Euromedex). Primary mouse anti-human Ki67 antibody (Dako) or primary rabbit anti-human cleaved Caspase-3 antibody (Abcam) was applied overnight. Next, skin slices were incubated for 1 hour at room temperature with goat-anti mouse or goat -anti rabbit IgG Alexa 555 (LifeTech) at 0.05% in PBS plus 0.1% DAPI 1000X (Sigma). Slices were mounted between slides and coverslips using Fluoromount (Sigma). 10 fields were acquired per sample using a DM5000 (Leica).

The number of cells in which both DAPI and KI67 signals colocalize and the percentage of the epidermis area which was positive for Caspase 3 was quantified using Image J.

Figure 9 A is the quantification of the number of proliferating cells in the epidermis of the *ex-vivo* skin explants issued from six different donors (1, 2, 3, 4, 5 and 6) and cultured over 10 days in conditions described in the examples, NativeSkin^{®} models in dark and FlowSkin^{®} models in grey. The number of proliferating cells is expressed as the percentage of proliferation measured the day of models' production. Each bar represents means ±SEM of proliferation measured for each condition. Figure 9 A shows that after 10 days of culture, the rate of epidermal cell proliferation is still relatively high for both NativeSkin^{®} (black bars) and FlowSkin^{®} (grey bars) models.

Figure 9 B is the quantification of the percentage of apoptosis in the epidermis of the *ex-vivo* skin explants issued from six different donors (1, 2, 3, 4, 5 and 6) and cultured over 10 days in conditions described in the examples, NativeSkin^{®} models in dark and FlowSkin^{®} models in grey. The percentage of apoptosis is assessed using an anti-cleaved Caspase-3 immunostaining. Each bar represents means ±SEM of apoptosis measured for each condition. Figure 9 B shows that the rate of apoptosis is low in the epidermis for both NativeSkin^{®} (black bars) and FlowSkin^{®} (grey bars) models.

### II.1.3 - Metabolic activity of skin cells

Metabolic activity of skin cells is measured by dosing glucose consumption and lactate secretion in the ex vivo skin models. Metabolites dosing was performed using the Glucose-Glo^{™} assay and the Lactate-Glo^{™} assay (Promega) following manufacturer's instructions. Metabolites were dosed in culture media of both NativeSkin^{®} (dark bars) and FlowSkin^{®} models (grey bars), sampled after 10 days of *ex-vivo* culture. Glucose consumption (Figure 10 A) is expressed in percentage of the initial glucose value in the medium, and lactate production (Figure 10 B) is expressed in µM.

Figure 10 shows that for both conditions (NativeSkin^{®} or FlowSkin^{®}), a high glucose consumption (figure 10 A) and a high lactate production (figure 10 B) is visible still after 10 days of culture. This shows that skin cells are still metabolically active for both FlowSkin^{®} (grey bars) and the NativeSkin^{®} (black bars) models.

All these results clearly establish that *ex-vivo* skin explants can be infused for up to 10 days while maintaining their integrity and viability.

### II.2 - Characterization of the diffusion in the FlowSkin^{®} model

In order to characterize diffusion in the FlowSkin^{®} model, a dye has been perfused through the implantable microfluidic device. The dye has been infused at a rate of 5 µl/min for 30 seconds, followed by 0 µl/min for 30 seconds on a cyclic basis for 24 hours. No pressure control is performed during this experiment.

Pictures of the ex-vivo skin models has been taken at different time points: 0, 2, 4, 7 and 24 hours of perfusion. At each time point, color intensity has been measured following an axis perpendicular to the catheter implantation axis, from one edge of the biopsy to the other. This color intensity is directly correlated to dye concentration in the biopsy.

As illustrated in Figure 11, a growing color intensity is visible at the surface of the FlowSkin^{®} model, reflecting an increasing concentration of dye in the tissue.

### III - Applications of the FlowSkin^{®} model

### III.1 - Simulation of the diffusion in the FlowSkin^{®} model

Figure 12 is a diffusion modeling in NativeSkin^{®} and FlowSkin^{®} models using COMSOL software. The graphs represent the evolution of the concentration of molecules of interest on a sectional view at the center of the *ex-vivo* skin explant, at times ranging from 0 to 24 hours of culture. A and B: NativeSkin^{®}. C and D: FlowSkin^{®}.

### III.1.1- Hypothesis

The diffusion coefficient in the skin is 1.26.10⁻⁶ cm²/s. The diffusion coefficient in the matrix is 1.1.10⁻⁵ cm²/s. The skin is homogeneous. The skin is the same from one donor to another. The volume of an *ex-vivo* skin explant of 15mm diameter is always the same.

A and C: Modeling the diffusion of a molecule of interest present both in the culture medium in the well under *ex-vivo* skin explant and in the perfusate at an arbitrary initial concentration set at 1, and in the matrix at a concentration of 0.4. This model can be equated to simulating the diffusion of nutrients present in the culture medium (as this culture medium is also present in the matrix).

B and D: Modeling the diffusion of a molecule of interest present only in the well under *ex-vivo* skin explant and in the perfusate at an arbitrary initial concentration set at 1. This model can be equated to simulating the diffusion of a drug administered systemically.

### III.1.2 - Results

As illustrated in figure 12 A and C, after 24 hours of infusion, the maximum concentration of nutrients reached in the *ex-vivo* skin explant is higher in the edges for the FlowSkin^{®} model (70% of the culture medium concentration) than for the NativeSkin^{®} model (60% of the culture medium concentration). Moreover, unlike in NativeSkin^{®} model wherein the concentration of nutrients is homogeneous both in the edges and in the center of the biopsy, a real gradient of concentration of nutrients occurs from center of the biopsy to the edges for the FlowSkin^{®} model.

The entire *ex-vivo* skin explant reaches a concentration of at least 50% of the culture medium concentration in 14 hours for the NativeSkin^{®} model compared to 12 hours for the FlowSkin^{®} model.

Similarly, as illustrated in figure 12 B and D, the maximum concentration of the molecule of interest reached in the *ex-vivo* skin explant is of 60% of the culture medium concentration for the FlowSkin^{®} model instead of 45% for the NativeSkin^{®} model.

### III.2 - Skin inflammation model

In order to evaluate the potential interest of the FlowSkin^{®} system to induce an inflammation in a healthy skin and to reduce inter donor variability observed with the InflammaSkin^{®} model, a comparison between the standard InflammaSkin^{®} model and the InflammaSkin^{®} model perfused using the FlowSkin^{®} system has been carried out. Models of both conditions have been activated by injection of an activation cocktail, and have then been cultured in culture medium supplemented with pro-inflammatory cytokines. These cytokines have also been added in the perfused culture medium for the perfused InflammaSkin^{®} model.

### III.2.1 - Activation of resident T cells

Using a clamp, the *ex-vivo* skin explant is held flat, with the dermis on top. A needle with no dead volume is inserted into the *ex-vivo* skin explant from the side of the dermis using a 05N Hamilton syringe35 µL of the activation solution (50 ng/µL anti-CD3 antibody, 50 ng/µL anti-CD28 antibody, and 10 ng/mL IL-2) are injected from the side of the biopsy to avoid damage to the epidermis.

The activation solution containing the mixture of anti-CD3, anti-CD28 and IL-2 at the same concentration could also be injected continuously for the first 24 hours thanks to the implanted microfluidic device of the FlowSkin^{®} model. Injection could be performed in recirculation for 24 hours, at the same flow-rate and cycle of perfusion than described in paragraph 7.

### III.2.2 - Differentiation of T cells into Th1/Th17

In WILLIAM'S E synthetic culture medium, the following supplements are added: 10 ng/mL of IL-1β, 50 ng/mL of IL-23, and 10 ng/mL of TGFβ. The culture is carried out for 7 days in a CO₂ incubator at 37 °C. The culture medium containing the supplements is changed daily for the *ex-vivo* skin explant of the NativeSkin^{®} model and of the FlowSkin^{®} model.

In addition, the culture medium containing the supplements is also injected continuously, using the microfluidic implantable device having pores of 50 µm diameter in its porous portion, in the *ex-vivo* skin explant of the FlowSkin^{®} model, at the same flow-rate and cycle of perfusion than described in paragraph 7

### III.2.3 - Production of inflammation markers

The cytokine IL-22 produced by the models were analyzed in the culture media by an ELISA assay (Human IL-22 Quantikine ELISA Kit, R&D Systems).

### III.2.4 - Results

Figure 12 shows the evolution of the concentration of IL-22 (pg/mL) released into the culture medium over time for two donors. For Donors 1 (D1) and 5 (D5), the concentration of IL-22 is higher in FlowSkin^{®} models (full and doted grey lines) as compared to NativeSkin^{®} models (full and doted black lines); the highest concentration of IL-22 being observed at day 2. This means that infusion of cytokine-supplemented culture medium improves the inflammatory response of the FlowSkin^{®} models, as compared to NativeSkin^{®} models.

In addition, the concentration of IL-22 released between D0 and D1 is higher in perfused FlowSkin^{®} models for donor 5 than in non-perfused NativeSkin^{®} models. Infusion improves cytokine diffusion during the first 24 hours of culture for donor 5.

### III.3 - Impact of oxygen carrier on long term culture

### III.3.1 - Culture conditions

Oxygen delivery is based on a 3.6 MDa marine macroparticule able to carry 156 molecules of oxygen when saturated. HemoxCell^{®} is a ready-to use solution that brings oxygen and releases it according to the PO2 gradient. HemoxCell^{®} is added in the culture media (William's E synthetic culture medium supplemented with 0.1 % CaCl₂ at 1mM, 1 % Penicillin/Streptomycin, 0.2 % Amphotericin B and 2 % Vitamin C at 10 mg/mL) at a concentration of 0.05 g/l.

A comparative study is performed on NativeSkin^{®} and FlowSkin^{®} models to assess the impact of oxygen carrier administered by passive diffusion (NativeSkin^{®}) or by intra-skin perfusion (FlowSkin^{®}) on physiology.

Twelve *ex-vivo* skin explants (6 NativeSkin^{®} and 6 FlowSkin^{®}) issued from 3 donors are prepared according to the method of the present invention or according to the method described in European patent EP 2882290 B1. The *ex-vivo* skin explants are 15 mm diameter sized, with 12/15 mm in diameter silicon rings. The liquid matrix capable of solidifying results from a mixture of a first solution of fibrinogen and tranexamic acid at a 10:1 ratio and a second solution of agarose 1.07%, 130 mM NaCl and 10 mM CaCl₂. The porous portion of the implantable microfluidic device contains pores having a diameter of 50 µm. The *ex-vivo* skin explants are cultured over 10 days in standard culture conditions in an incubator at 37°C, 5% CO₂.

For NativeSkin^{®} model, the 2 ml of culture medium containing HemoxCell^{®} is renewed every day. For FlowSkin^{®} model, culture medium containing HemoxCell^{®} is perfused cyclically at a flow rate of 5µl/min for 30 seconds and 0 µl/min for 30 seconds.

### III.3.2 - Sampling

At each time points (D0 and D10 days), *ex-vivo* skin explants are carefully unmolded and cut in half, perpendicularly to catheter implantation axis in the case of the FlowSkin^{®} models. One half is fixed in 10% buffered formalin for 48 hours at room temperature, to perform histological analysis. Three punches of 2mm in diameter are produced in the other half to perform WST-8 assay. The leftover is frozen at -80°C for further analysis.

Culture media is sampled and frozen at -80°C for further analysis.

### III.3.3 - Evaluation of cell metabolism and skin structure integrity

WST-8 assay is performed on 2mm in diameter fresh *ex-vivo* skin explants according to protocol described in paragraph 7.1. WST-8 solutions of the D0 control are frozen at - 20°C until reading with the D10 samples. Absorbance is read using a microplate reader (Victor Nivo, Perkin Elmer). Formalin fixed skin explant are dehydrated and paraffin-embedded. 5µm thickness skin cross-sections are sliced using a microtome.

Hematoxylin and Eosin staining is performed on 5µm skin cross-sections according to conditions described in paragraph 7.1. Images are taken using a Transmitted-Light microscope (DMi1, Leica) equipped with a MC170 HD camera.

Anti-Ki67 immunostaining is performed on 5µm skin cross-sections by using mouse anti-Ki67 monoclonal antibody (DAKO M724029) at a dilution of 1/500 and goat anti-mouse secondary antibody (Life Tech A21235) at a dilution of 1/500. Images are taken using an Axiolmager M2.

Anti-cleaved Caspase-3 immunostaining is performed on 5µm skin cross-sections by using rabbit anti-cleaved Caspase-3 polyclonal antibody (Abcam ab23020) at a dilution of 1/100 and goat anti-rabbit secondary antibody (Life Tech A21244) at a dilution of 1/500. Images are taken using an Axiolmager M2.

## Claims

1. An *in-vitro* method for culturing an *ex-vivo* skin explant comprising:
(a) inserting an implantable microfluidic device through the dermis or hypodermis of an ex-vivo skin explant so that the device is positioned substantially parallel to the epidermis and traverses the skin explant from side to side, both ends of the device protruding slightly beyond the skin explant,
(b) connecting the two ends of the implantable microfluidic device to two separate tubings,
(c) immersing the skin explant obtained in step (b) in a liquid matrix capable of solidifying so that the upper surface of the epidermis is not covered, which matrix is itself contained in a cell culture insert, the bottom of which consists of a porous membrane,
(d) solidifying the matrix so as to trap the immersed part of the skin explant, where the upper surface of the epidermis is not covered, and to cause the solidified matrix to adhere to the side walls and the porous membrane of the insert,
(e) putting the culture insert containing the skin explant obtained in step (d) in a culture chamber containing appropriate culture medium, and
(f) culturing the skin explant.

2. The method according to claim 1, wherein the portion of the implanted microfluidic device that passes through the ex-vivo skin explant is porous.

3. The method according to claim 2, wherein the porous portion of the implantable microfluidic device is obtained by making pores having a diameter from about 40 µm to about 250 µm, more preferably from about 50 µm to about 200 µm, most preferably of about 50 µm.

4. The method according to any one of claims 1 to 3, further comprising a step of:
(g) connecting the tubings to a circulating system comprising means for providing a continuous or discontinuous/pulsatile flow and/or for modulating the difference of pressure between the inlet and the outlet of the implantable device, said connecting being mediated by input and output ports at the inlet and outlet of the implantable device.

5. The method according to claim 4, wherein the flow rate of a fluid perfused within the *ex-vivo* skin explant ranges from about 0 µl/minute to 1000 µl/minute, preferably about 1 µl/minute to 20 µl/minute, more preferably from about 2.5 µl/minute to 10 µl/minute, and most preferably from about 2.5 µl/minute to 5 µl/minute.

6. The method according to claim 4, wherein the difference of pressure accross the implantable device ranges from about -1 bar to 1 bar, preferably from about - 200 mBar to 200 mbar and even more preferably from about -50 mbar to 50 mbar

7. The method according to any one of claims 1 to 6, wherein said method is for further administering a compound of interest selected from the group comprising or consisting of culture media, oxygen carrier or a drug within the ex-vivo skin explant, said method comprising a step of injecting said compound via the circulating system.

8. The method according to any one of claims 1 to 6, wherein said method is for further detecting and/or quantifying at least one biomarker of interest contained in the liquid secreted by the *ex-vivo* skin explant, and said method further comprises the following steps:
(h) collecting the liquid secreted by the *ex-vivo* skin explant through the output port, and
(i) isolating, identifying, and optionally quantifying the at least one biomarker of interest contained in the collected fractions of the liquid secreted by the *ex-vivo* skin explant.

9. The method according to any one of claims 1 to 6, wherein said method is for further assessing permeation of the at least one biomarker of interest though the skin, said method further comprises a step (g') of applying topically to the epidermis of the *ex-vivo* skin explant a composition comprising the at least one biomarker of interest after the step (g) of connecting the tubings to a circulating system and prior to the step (h) of collecting the liquid secreted by the *ex-vivo* skin explant.

10. The method according to any one of claims 1 to 6, wherein said method is for further assessing the activity of at least one compounds intradermally injected, said method further comprises a step (g") of injecting a composition comprising the at least one compound of interest after the step (g) of connecting the tubings to a circulating system and prior to the step (h) of collecting the liquid secreted by the *ex-vivo* skin explant, the composition being injecting in the dermis of the *ex-vivo* skin explant by using the microfluidic implantable device.

11. The method according to any one of claims 1 to 6, wherein said method is for further assessing the activity of at least one compounds subcutaneously injected, said method further comprises a step (g'") of injecting a composition comprising the at least one compound of interest after the step (g) of connecting the tubings to a circulating system and prior to the step (h) of collecting the liquid secreted by the *ex-vivo* skin explant, the composition being injecting in the hypodermis of the *ex-vivo* skin explant by using the microfluidic implantable device.

12. The method according to any one of claims 1 to 6, wherein said method is for further oxygenating the *ex-vivo skin* explant, said method further comprises a step (g"") of injecting an oxygen carrier after the step (g) of connecting the tubings to a circulating system.

13. The method according to any one of claims 1 to 6, wherein said method is for further studying inflammation of the skin, said method further comprises a step (g'"") of injecting a composition comprising a cocktail of cytokines after the step (g) of connecting the tubings to a circulating system and prior to the step (h) of collecting the liquid secreted by the *ex-vivo* skin explant, the composition being injecting in the dermis or hypodermis of the *ex-vivo* skin explant by using the microfluidic implantable device.

14. A system comprising a cell culture insert the bottom of which consists of a porous membrane, said culture insert containing an *ex-vivo* skin explant embedded in a solidified matrix, wherein the *ex-vivo* skin explant comprises the epidermis, the dermis and optionally the hypodermis **characterized in that** an implantable microfluidic device is inserted through the dermal or hypodermal part of the ex-*vivo* skin explant, wherein the microfluidic device is positioned substantially parallel to the epidermis and traverses the *ex-vivo* skin explant from side to side, both ends of the device protruding slightly beyond the *ex-vivo* skin explant.

15. Use of the system of claim 14 for:
- identifying percutaneous penetration of potentially dangerous exogenous agents from the environment,
- administering high molecular weight molecules in a time-controlled manner,
- studying the diffusion of an infused compound into the dermis or hypodermis,
- comparing different routes of administration,
- testing different modes of administration (over time),
- studying skin inflammation,
- assessing toxicity of drugs administered by subcutaneous route,
- assessing bioavailability of a cosmetic agent administered by topical route,
- assessing permeation rate of a compound administered by topical route, or
- obtaining long term culture of *ex-vivo* skin explant.

16. A device for inserting a microfluidic implantable device into an *ex-vivo* skin explant, said device comprising two separate and removable stackable parts which, one assembled together, form:
(a) a central cavity suitable for receiving and maintaining an *ex-vivo* skin explant, and
(b) ducts through which a microfluidic implantable device is guided to go through the *ex-vivo* skin explant from side to side.

17. A method for inserting a microfluidic implantable device in an *ex-vivo* skin explant, comprising the steps of:
(a) placing the skin explant in the recess of the first stackable part of the device as defined in claim 15, where the epidermis is in contact with this first element and the dermis or hypodermis is in contact with the air,
(b) assembling the second stackable part of the device on the first stackable part containing the skin explant so that the dermis or hypodermis of the skin explant is in contact with the recess of the second stackable part of the device,
(c) fastening the two stackable parts of the device comprising the skin explant,
(d) introducing the implantable microfluidic device in the groove,
(e) passing the implantable microfluidic device through the skin explant from side to side so as to position the porous part of the implantable microfluidic device is located in the center of the skin explant.
